(19) Europäisches Patentamt
European Patent Office
Office européen des brevets

(11) Publication number: **0 406 473 B1**

(12) # EUROPEAN PATENT SPECIFICATION

(45) Date of publication of patent specification: **20.09.95**

(51) Int. Cl.6: **G01N 33/538**, G01N 33/543

(21) Application number: **89114103.8**

(22) Date of filing: **31.07.89**

Divisional application 95102406.6 filed on 21/02/95.

(54) **Ion capture reagents and methods for performing binding assays.**

(30) Priority: **07.07.89 US 375029**

(43) Date of publication of application:
**09.01.91 Bulletin 91/02**

(45) Publication of the grant of the patent:
**20.09.95 Bulletin 95/38**

(84) Designated Contracting States:
**AT BE CH DE ES FR GB IT LI NL**

(56) References cited:
**EP-A- 0 230 768**
**EP-A- 0 245 926**
**EP-A- 0 268 296**
**EP-A- 0 326 100**

(73) Proprietor: **ABBOTT LABORATORIES**
**CHAD-0377,**
**AP6D/2,**
**One Abbott Park Road**
**Abbott Park,**
**Illinois 60064-3500 (US)**

(72) Inventor: **Hiltibran Robert G.**
**8214 200th Avenue**
**Bristol, WI 53104 (US)**
Inventor: **Stroupe, Stephen Denham**
**606 Roosevelt**
**Libertyville, IL 60048 (US)**
Inventor: **Jou, Yi-Her**
**102 Austin Court**
**Vernon Hills, IL 60061 (US)**

(74) Representative: **Modiano, Guido, Dr.-Ing. et al**
**Modiano & Associati S.r.l.**
**Via Meravigli, 16**
**I-20123 Milano (IT)**

**EP 0 406 473 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Description for the following Contracting States : BE, CH, DE, ES, FR, GB, IT, NL

### BACKGROUND OF THE INVENTION

1. Field of the Invention

This invention relates generally to the field of binding assay devices and methods. In particular, the present invention relates to novel methods useful in the performance of homogeneous immunoassays.

2. Description of Related Art

Various analytical procedures and devices are commonly employed in assays to determine the presence and/or concentration of substances of interest or clinical significance which may be present in biological liquids or other materials. Such substances are commonly termed "analytes" and can include antibodies, antigens, drugs, hormones, etc.

Immunoassay techniques take advantage of the mechanisms of the immune systems of higher organisms, wherein antibodies are produced in response to the presence of antigens which are pathogenic or foreign to the organisms. These antibodies and antigens, i.e,, immunoreactants, are capable of binding with one another, thereby creating a highly specific reaction mechanism which can be used *in vitro* to determine the presence or concentration of that particular antigen in a biological sample.

There are several known immunoassay methods using immunoreactants, wherein at least one of the immunoreactants is labeled with a detectable component so as to be analytically identifiable. For example, the "sandwich" or "two-site" technique may involve the formation of a ternary complex between an antigen and two antibodies. A convenient method of detecting complex formation in such a technique is to provide one labeled antibody and an unlabeled antibody bound to a solid phase support such that the complex can readily be isolated. In this example, the amount of labeled antibody associated with the solid phase is directly proportional to the amount of analyte in the test sample.

An alternative technique is the "competitive" assay. In one example of a competitive assay, the capture mechanism again may use an antibody attached to an insoluble solid phase. but a labeled analyte (rather than a labeled antibody) competes with the analyte present in the test sample for binding to the immobilized antibody. Similarly, an immobilized analyte can compete with the analyte of interest for a labeled antibody. In these competitive assays, the quantity of captured labeled reagent is inversely proportional to the amount of analyte present in the sample.

Despite their great utility, there are disadvantages with such assay methods. First, the heterogenous reaction mixture of soluble and insoluble reagents, and liquid test sample, can retard the kinetics of the reaction. In comparison to a liquid phase reaction wherein all reagents are soluble, i.e. a homogeneous reaction mixture, the heterogenous reaction mixture can require longer incubation periods for equilibrium to be reached in the reaction mixture between the insoluble solid phase system, the free analyte in the test sample, the soluble labeled reagent, and the newly formed insoluble complex. Second, conventional methods of attaching binding members to the solid phase material, such as adsorption of antibody to the solid phase, can produce a solid phase which will readily bind substances other than the analyte. This is referred to as nonspecific binding and can interfere with the detection of a positive result. Third, with conventional immobilization methods, separate batches of manufactured solid phase reagents can contain variable amounts of immobilized binding member.

Of interest as background art to the present invention is EP-A-0 245 926 disclosing methods of measuring antigens or antibodies in biological fluids relying on the separation of complexes by means of specific binding phenomena.

Likewise, EP-A- 0 268 296 discloses immunoassay methods relying on the separation of complexes by means of specific binding phenomena.

Also of interest is EP-A-0 326 100 which discloses ion-capture assays and separation methods. EP-A-0 326 100 is considered state of the art pursuant to Article 54(3) EPC and is therefore only relevant for the assessment of novelty.

Of further interest is EP-A-0 230 768 which discloses a specific binding assay involving coagglutination of a non-magnetic particle, to which is bound a capture reagent for the analyte, and a solid phase comprising magnetic particles. The coagglutination occurs by means of ionic interaction of the particles. The assay medium is subjected to a magnetic field gradient to separate the coagglutinated particles from

the medium. After the separation, the medium or the particles are examined for the presence or amount of a specific binding member, which is affected by the presence of analyte in the sample.

## SUMMARY OF THE INVENTION

The present invention provides a method for detecting an analyte in a test sample, comprising the steps of:

a) contacting the sample with

a soluble capture reagent, comprising a hapten conjugated to a first charged substance, and

an indicator reagent, comprising a specific binding member conjugated to a label capable of producing a detectable signal, thereby forming a reaction mixture,

wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,

b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and

c) detecting said label associated with said solid phase or said reaction mixture as an indication of the presence or amount of the analyte in the sample.

The present invention also provides a method for detecting an analyte in a test sample, comprising the steps of:

a) contacting the sample with

a soluble capture reagent, comprising a specific binding member conjugated to a first charged substance, and

an indicator reagent, comprising a hapten conjugated to a label capable of producing a detectable signal, thereby forming a reaction mixture,

wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,

b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and

c) detecting said label associated with said solid phase or said reaction mixture as an indication of the presence or amount of the analyte in the sample,

provided that competitive assays wherein the indicator reagent and analyte compete in binding to the capture reagent are excluded.

The present invention provides methods using capture reagents which facilitate the observation of a detectable signal by separating the analyte and/or indicator reagent from the other assay reagents or test sample. The capture reagents may comprise one or more anionic molecules attached to a specific binding member. A readily adapted anionic molecule is an activated polymeric anionic molecule having the formula:

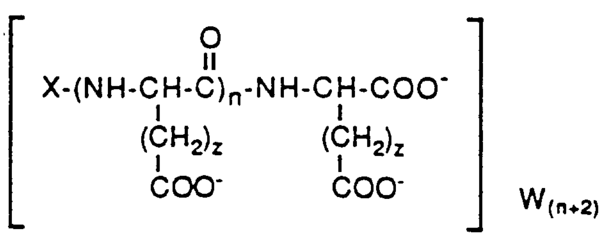

wherein n is about 10 to about 500; z is about 1 to about 6; W is chosen from $H^+$, $Na^+$, $K^+$, $Li^+$, amine salts such as $H^+NR_3$, and derivatives thereof; and X is an amine-reactive moiety, a thiol-reactive moiety or a thiol moiety with which the specific binding member will react. Alternatively, X can represent a specific binding member which has been activated to bind the polymeric anionic molecule. Activation methods are also described by which one or more reactive groups are formed upon the specific binding member or the polymeric anion.

The specific binding member component of the capture reagent can be either a hapten or a macromolecule. The charged capture reagent enables homogeneous assay and separation reactions wherein the reaction complexes can be removed from the reaction mixture by contacting the mixture with an oppositely charged solid phase. Virtually any binding assay of the invention (sandwich assays, competitive assays, indirect assays using ancillary specific binding members, inhibition assays, etc.) can be carried out using the above capture reagents.

The present invention brings two advantages to binding assays: a) the use of liquid phase kinetics in the binding reaction facilitates the formation of a complex from the homogeneous mixture of analyte and assay reagents, and b) it increases the potential number of complexes that can be immobilized on the solid support.

If liquid phase kinetics are not sought, the present invention also provides an efficient method of immobilizing binding members on a solid phase through a method other than absorption, adsorption or covalent binding.

## DETAILED DESCRIPTION OF THE INVENTION

The assay methods of the present invention can be used in a variety of immunoassay formats. The present invention, however, is not limited to immunoreactive assays. Any assays using specific binding reactions between the analyte and assay reagents can be performed.

### Definitions

The following definitions are applicable to the present invention.

The term "specific binding member', as used herein, refers to a member of a specific binding pair, i.e., two different molecules where one of the molecules through chemical or physical means specifically binds to the second molecule. In addition to antigen and antibody-specific binding pairs, other specific binding pairs include biotin and avidin, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, hormone and hormone binding protein, enzyme cofactors and enzymes, enzyme inhibitors and enzymes, and the like. Furthermore, specific binding pairs can include members that are analogs of the original specific binding member. For example, a derivative or fragment of the analyte, i.e., an analyte-analog, can be used so long as it has at least one epitope in common with the analyte. Immunoreactive specific binding members include antigens, haptens, antibodies, and complexes thereof including those formed by recombinant DNA methods or peptide synthesis. An antibody can be a monoclonal or polyclonal antibody, a recombinant protein or a mixture(s) or fragment(s) thereof, as well as a mixture of an antibody and other specific binding members. The details of the preparation of such antibodies and their suitability for use as specific binding members are well known to those skilled in the art.

The term "hapten", as used herein, refers to a partial antigen or non-protein binding member which is capable of binding to an antibody, but which is not capable of eliciting antibody formation unless coupled to a carrier protein.

The term "test sample", as used herein, refers to virtually any liquid sample. The test sample can be derived from any desired source, such as a physiological fluid, for example, blood, saliva, ocular lens fluid, cerebral spinal fluid, sweat, urine, milk, ascites fluid, mucous, synovial fluid, peritoneal fluid, amniotic fluid or the like. The liquid test sample can be pretreated prior to use, such as preparing plasma from blood, diluting viscous liquids, or the like; methods of treatment can also involve separation, filtration, distillation, concentration, inactivation of interfering components, and the addition of reagents. Besides physiological fluids, other liquid samples such as water, food products and the like can be used. In addition, a solid can be used once it is modified to form a liquid medium.

"Analyte", as used herein, is the substance to be detected in or separated from the test sample using the present invention. The analyte can be any substance for which there exists a naturally occurring specific

binding member or for which a specific binding member can be prepared. In addition, the analyte may bind to more than one specific binding member. "Analyte" also includes any antigenic substances, haptens, antibodies, and combinations thereof. The analyte can include a protein, a peptide, an amino acid, a hormone, a steroid, a vitamin, a drug including those administered for therapeutic purposes as well as those administered for illicit purposes, a bacterium, a virus, and metabolites of or antibodies to any of the above substances.

The term "analyte-analog", as used herein, refers to a substance which cross-reacts with an analyte-specific binding member, although it may do so to a greater or a lesser extent than does the analyte itself. The analyte-analog can include a modified analyte as well as a fragmented or synthetic portion of the analyte molecule so long as the analyte-analog has at least one epitopic site in common with the analyte of interest.

The term "label", as used herein, refers to any substance which is attached to a specific binding member and which is capable of producing a signal that is detectable by visual or instrumental means. Various suitable labels for use in the present invention can include chromogens; catalysts; fluorescent compounds; chemiluminescent compounds; radioactive labels; direct visual labels including colloidal metallic and non-metallic particles, dye particles, enzymes or substrates, or organic polymer latex particles; liposomes or other vesicles containing signal producing substances; and the like.

A large number of enzymes suitable for use as labels are disclosed in U.S. Patent No. 4,275,149, columns 19-23. An example of an enzyme/substrate signal producing system useful in the present invention is the enzyme alkaline phosphatase and the substrate nitro blue tetrazolium-5-bromo-4-chloro-3-indolyl phosphate or a derivative or analog thereof.

In an alternative signal producing system, the label can be a fluorescent compound where no enzymatic manipulation of the label is required to produce a detectable signal. Fluorescent molecules such as fluorescein, phycobiliprotein, rhodamine and their derivatives and analogs are suitable for use as labels in this reaction.

In an especially preferred embodiment, a visually detectable, colored particle can be used as the label component of the indicator reagent, thereby providing for a direct colored readout of the presence or concentration of the analyte in the sample without the need for further signal producing reagents. Materials for use as the colored particles are colloidal metals, such as gold, and dye particles as disclosed in U.S. Pat. Nos. 4,313,734 and 4,373,932. The preparation and use of non-metallic colloids, such as colloidal selenium particles, are disclosed in EP-A-0 298 368 published on January 11, 1989 (co-owned and copending U.S. Patent Application Serial No. 072,084, filed July 9, 1987). The use of colloidal particle labels in immunochromatography is disclosed in EP-A-0 299 428 published on January 18, 1989 (co-owned and copending U.S. Patent Application Serial No. 072,459, filed July 13, 1987). Organic polymer latex particles for use as labels are disclosed in EP-A-0 360 088 published on March 28, 1990 (co-owned and copending U.S. Patent Application Serial No. 248,858, filed September 23, 1988).

A "signal producing component", as used herein, refers to any substance capable of reacting with another assay reagent or the analyte to produce a reaction product or signal that indicates the presence of the analyte and that is detectable by visual or instrumental means. "Signal production system", as used herein, refers to the group of assay reagents that are needed to produce the desired reaction product or signal. For example, one or more signal producing components can be used to react with a label and generate the detectable signal, i.e., when the label is an enzyme, amplification of the detectable signal is obtained by reacting the enzyme with one or more substrates or additional enzymes to produce a detectable reaction product.

An "indicator reagent", as used herein, refers to a label attached to a specific binding member. The indicator reagent produces a detectable signal at a level relative to the amount of an analyte in the test sample. Generally, the indicator reagent is detected or measured after it is captured on the solid phase material, but the unbound indicator reagent can also be measured to determine the result of an assay.

The specific binding member of the indicator reagent is capable of binding either to the analyte as in a sandwich assay, to the capture reagent as in a competitive assay, or to an ancillary specific binding member as in an indirect assay. The label, as described above, enables the indicator reagent to produce a detectable signal that is related to the amount of analyte in the test sample. The specific binding member component of the indicator reagent enables the indirect binding of the label to the analyte, to an ancillary specific binding member or to the capture reagent. The selection of a particular label is not critical, but the label will be capable of generating a detectable signal either by itself, such as a visually detectable signal generated by colored organic polymer latex particles, or in conjunction with one or more additional signal producing components, such as an enzyme/substrate signal producing system. A variety of different indicator reagents can be formed by varying either the label or the specific binding member; it will be

appreciated by one skilled in the art that the choice involves consideration of the analyte to be detected and the desired means of detection.

A "capture reagent", as used herein, refers to an unlabeled specific binding member attached to a charged substance. The attachment of the components is essentially irreversible and can include covalent mechanisms. The capture reagent is used to facilitate the observation of the detectable signal by substantially separating the analyte and/or the indicator reagent from other assay reagents and the remaining test sample. The specific binding member can be a small molecule, such as a hapten or small peptide, so long as the attachment to the charged substance does not interfere with the binding members binding site.

The specific binding member of the capture reagent is specific either for the analyte as in a sandwich assay, for the indicator reagent or analyte as in a competitive assay, or for an ancillary specific binding member, which itself is specific for the analyte, as in an indirect assay. The charged substance can include anionic and cationic monomers or polymers. For example, anionic polymers include polyglutamic acid (PGA), anionic protein or derivitized protein such as albumin, anionic polysaccharides such as heparin or alginic acid, polyaspartic acid, polyacrylic acid, and polyamino acids having a net negative charge at an appropriate pH (such as a pH in the range of 4 to 10.) Furthermore, the specific binding member can be joined to more than one charged monomer or polymer to increase the net charge associated with the capture reagent.

In one embodiment of the present invention, a negatively charged capture reagent can be prepared by conjugating a specific binding member to one or more activated polymeric anionic molecules and conjugate bases thereof represented by the general formula:

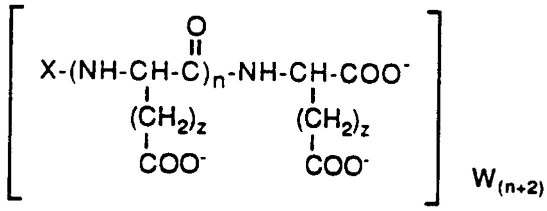

wherein n is 10 to 500; z is 1 to 6; W is chosen from $H^+$, $Na^+$, $K^+$, $Li^+$, amine salts such as $H^+NR_3$, and derivatives thereof; and X is virtually any reactive group or moiety having a reactive group that enables the chemical binding of the specific binding member and the polymer. X can be an amine-reactive group or moiety, a thiol-reactive group or moiety, or a thiol group or moiety represented by -A-SH wherein A is a spacer arm. For example, a specific binding member having an amino group can be conjugated to an activated PGA anionic molecule having an amine-reactive moiety. The amine-reactive moieties enable the binding of the activated polymer to an amino group on a specific binding member and include, but are not limited to, those represented by the following formulas

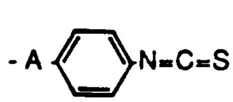 , 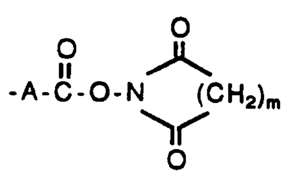 , 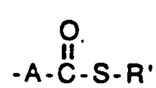 , 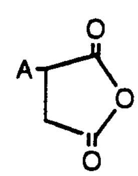 ,

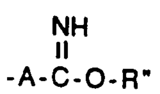 , 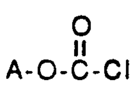 , 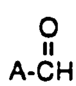 and the addition salts of 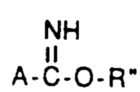

wherein m is two or three, R' is a sulfur stabilizer and R" is an aliphatic or aryl group. Sulfur stabilizers include, but are not limited to, 2-pyridyl, 4-pyridyl and 5-nitro-2-pyridyl groups. "A" represents a spacer of one to thirty atoms including, but not limited to, carbon, nitrogen, sulfur and oxygen atom chains and combinations thereof such as polyether, polymethylene and polyamide, as well as aromatic spacers such as phenylthiocarbamyl.

Alternatively, a specific binding member having a thiol group can be conjugated to an activated polymer having a thiol-reactive moiety. The thiol-reactive moieties include, but are not limited to, those represented by the following formulas

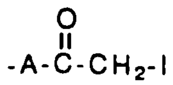 , 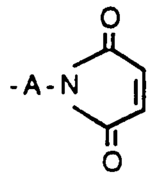 , 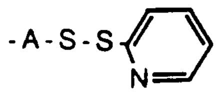 , 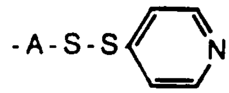 ,

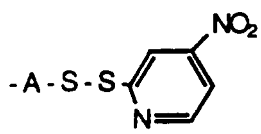 , 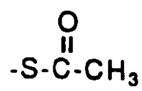 and 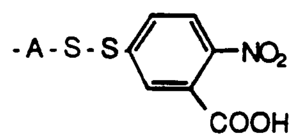

wherein A is a spacer of one to thirty atoms as described above. In yet another alternative, a specific binding member having a thiol-reactive group can be linked to an activated polymer having a thiol moiety such as -A-SH.

Typically, the negatively charged capture reagents of the following Examples were formed by reacting the desired specific binding member with an activated PGA molecule having modified terminal amino groups. Briefly, the modification method involved: 1) dissolving the PGA in a solvent (e.g., a water miscible aprotic solvent such as dioxane, dimethylformamide, 1-methyl-2-pyrrolidinone and dimethyl sulfoxide); 2) adding a proton absorbing reagent (e.g., 4-methyl morpholine)in the amount of about one equivalent per titratable carboxylic acid; 3) adding a 2 to 100 molar excess of an amine-reactive modification reagent (e.g., 1,4-phenylene diisothiocyanate dissolved in dimethylformamide); 4) reacting the mixture; and 5) removing the unreacted amine-reactive modification reagent. Suitable proton absorbing reagents include alkali metal hydroxides such as sodium hydroxide, potassium hydroxide or lithium hydroxide, and tertiary amines such as 4-methyl morpholine and triethylamine.

The polymeric anionic molecule or the specific binding member will include one or more amino, carboxyl or thiol groups or can be activated by the incorporation of an amino, carboxy! or thiol group thereby enabling the chemical cross-linking of the specific binding member with the polymeric anionic molecule. "Activated species" refer to specific binding members and polymeric anionic molecules which contain a reactive group through the incorporation of a cross-linking or other activating agent. The amine-reactive modification reagents are a subclass of those reagents used to "activate" a specific binding member or polymeric anionic molecule, i.e., to prepare the specific binding member or the polymeric anionic molecule for chemical cross-linking. Activating agents also include thiol introducing agents such as the thiolanes (such as 2-iminothiolane), succinimidyl mercaptoacetates (such as N-succinimidyl-S-acetylmercaptoacetate), and disulfide compounds which are subsequently reduced to a thiol. The thiol introducing agents can be used to activate specific binding members and solid phase materials for their subsequent reaction with a thiol-reactive group.

Amine-reactive modification reagents include, but are not limited to, bifunctional crosslinking or coupling agents, such as succinic anhydride analogs, iminothiolane analogs, homobifunctional reagents and heterobifunctional reagents, which enable the chemical cross-linking of the specific binding member and the polymeric anionic molecule. Examples of homobifunctional reagents can be represented by the formula X-A-X wherein X is an amine-reactive group and A is a spacer of one to thirty atoms. Examples of heterobifunctional reagents can be represented by the formula X-A-Y, wherein X is an amine-reactive group, Y is a thiol-reactive moiety, a thiol moiety or a thiol precursor and A is a spacer of one to thinly atoms as described above. Proteinaceous specific binding members with cysteine residues at the protein's active site can have their activity decreased by the addition of a coupling agent, therefore the cysteine residues in the active site must be protected, by means known in the art, prior to reacting the protein with the coupling agent.

The term "coupling agent", as used herein, includes bifunctional crosslinking or coupling agents, i.e., molecules containing two reactive groups or "ends", which may be tethered by a spacer. The reactive ends can be any of a variety of functionalities including, but not limited to: amino reacting ends such as N-hydroxysuccinimide (NHS) active esters, imidoesters, aldehydes, epoxides, sulfonyl halides, isocyanate,

isothiocyanate, and nitroaryl halides; and thiol reacting ends such as pyridyl disulfides, maleimides, thiophthalimides, and active halogens. The heterobifunctional crosslinking reagents have two different reactive ends, e.g., an amino-reactive end and a thiol-reactive end, while homobifunctional reagents have two similar reactive ends, e.g., *bis*maleimidohexane (BMH) which permits the cross-linking of sulfhydryl-containing compounds, and NHS homobifunctional crosslinkers such as disuccinimidyl suberate (DSS) as well as the water soluble analogs, sulfo-NHS esters (Pierce 1989 Handbook and General Catalog; Pierce, Rockford, IL, 61105-9976).

Other commercially available homobifunctional cross-linking reagents include, but are not limited to, the imidoesters such as dimethyl adipimidate dihydrochloride (DMA); dimethyl pimelimidate dihydrochloride (DMP); and dimethyl suberimidate dihydrochloride (DMS). The iminothiolane analogs can be represented by the general formula:

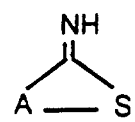

wherein A is a spacer of 1 to 5 atoms, e.g., 2-iminothiolane (Traut's Reagent.)

Commercially available heterobifunctional reagents suitable for use in the present invention include, but are not limited to, maleimido-NHS active esters coupling agents such as *m*-maleimidobenzoyl-N-hydroxysuccinimide ester (MBS); succinimidyl 4-(N-maleimidomethyl)cyclohexane-1-carboxylate (SMCC); succinimidyl 4-(*p*-maleimidophenyl)butyrate (SMPB) and derivatives thereof, including sulfosuccinimidyl derivatives such as sulfosuccinimidyl 4-(N-maleimido-methyl) cyclohexane-1-carboxylate (sulfo-SMCC); *m*-maleimidobenzoyl-sulfosuccinimide ester (sulfo-MBS) and sulfosuccinimidyl 4-(*p*-maleimidophenyl)butyrate (sulfo-SMPB).

Other suitable heterobifunctional reagents include commercially available active halogen-NHS active esters coupling agents such as N-succinimidyl bromoacetate and N-succinimidyl(4-iodoacetyl)-aminobenzoate (SIAB) and the sulfosuccinimidyl derivatives such as sulfosuccinimidyl(4-iodoacetyl)-aminobenzoate (sulfo-SIAB). Another group of coupling agents is the heterobifunctional and thiol cleavable agents such as N-succinimidyl 3-(2-pyridyldithio)propionate (SPDP).

Yet another group of coupling agents includes the extended length heterobifunctional coupling agents described in EP-A-0 314 127, published on May 3, 1989 (co-owned and copending U.S. Patent Application Serial Number 114,930, filed October 30, 1987). The extended length heterobifunctional coupling agents include maleimido-NHS active ester reagents wherein the spacer is represented by the formula:

$$-\ (\text{amino acid})_n - \overset{\displaystyle O}{\overset{\|}{C}} - R -$$

wherein the amino acid is a substituted or unsubstituted amino acid, having from three to ten carbon atoms in a straight chain; n is from one to ten; and R is an alkyl, cycloalkyl, alkyl-cycloalkyl or an aromatic carboxylic ring. The term alkyl-cycloalkyl includes alkyl groups linked to cycloalkyl ring structures where the alkyl group links the cycloalkyl to a maleimide or carbonyl group. The term alkyl includes straight or branched alkyl groups, preferably lower alkyl groups having from one to six carbon atoms.

If a spacer is present, the spacer can be any molecular chain that is non-reactive, stable and non-binding to the analyte or other specific binding members with which it will be used. The length of the spacer can be varied and can range from the size of a single atom to the sizes disclosed in U.S. Patent Applications Serial Numbers 254,288 and 114,930 or larger.

The choice of the amine-reactive modification reagent, thiol introducing agent or other activating agent is not critical, but one skilled in the art will know of suitable or preferred agents for use with the particular polymeric anionic molecule and specific binding member to be used in the diagnostic assay. Therefore, it will be appreciated by those skilled in the art that the coupling agent or activating agent used in a given assay will generally be determined empirically.

Suitable thiol-reactive moieties of the heterobifunctional reagents include, but are not limited to, those represented by the following formulas:

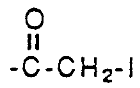 , 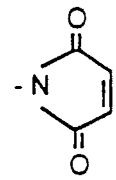 , 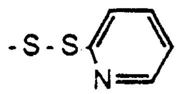 , 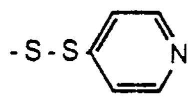 ,

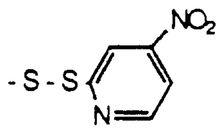 , and 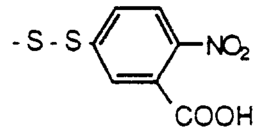

Suitable thiol precursor moieties include, but are not limited to, those represented by the following formulas:

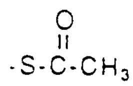 , 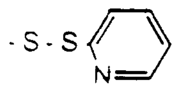 , 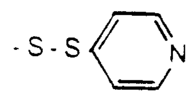 ,

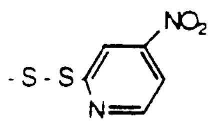 , and 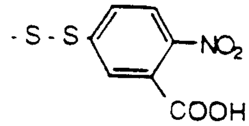

Suitable amine-reactive moieties include, but are not limited to, those represented by the following formulas:

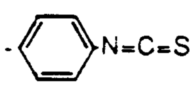 , 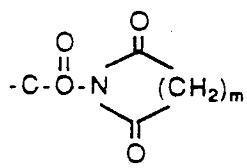 , 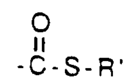 , 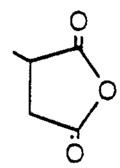 ,

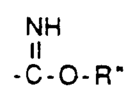 , 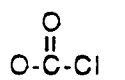 , 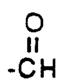 , and the addition salts of 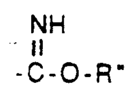 ,

wherein m is 2 or 3, R' is a sulfur stabilizer, as described above, and R" is an aliphatic or aryl group.

In yet another embodiment of the present invention, a specific binding member having an amine-reactive group (e.g., an activated specific binding member) can be conjugated to a terminal amino group of the polymeric anionic molecule. Briefly, an example of a conjugation procedure involves: 1) dissolving PGA in a solvent (e.g., a water miscible aprotic solvent such as dioxane, dimethylformamide, 1-methyl-2-pyrrolidinone and dimethyl sulfoxide); 2) adding a proton absorbing reagent (e.g., an alkali metal hydroxide such as sodium hydroxide, potassium hydroxide, or lithium hydroxide, or a tertiary amine such as 4-methyl morpholine or triethylamine) in the amount of about one equivalent per titratable carboxylic acid; 3) adding a 2 to 100 molar excess of amine-reactive specific binding member (e.g., phosgen-activated phenylcyclidine or phenylcyclidine-4-chloroformate); 4) reacting the mixture and 5) removing the unreacted amine-reactive specific binding member. Suitable examples of amine-reactive groups on specific binding members include, but are not limited to,

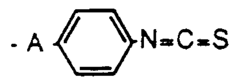, 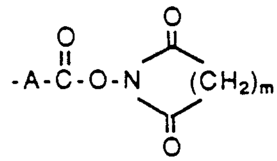, 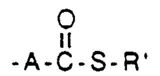, 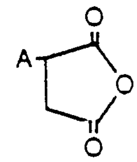,

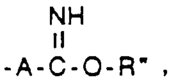, 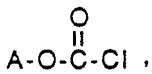, 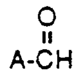 and the addition salts of 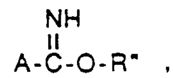,

wherein A is a spacer of about one to about thirty atoms as described above, m is two or three, R' is a sulfur stabilizer and R" is an aliphatic or aryl group.

An example of the preparation of a negatively charged capture reagent involves the reaction of a specific binding member (SBM) having an amino group and an activated PGA having an amine-reactive moiety. The resulting reaction and reaction product can be illustrated as follows:

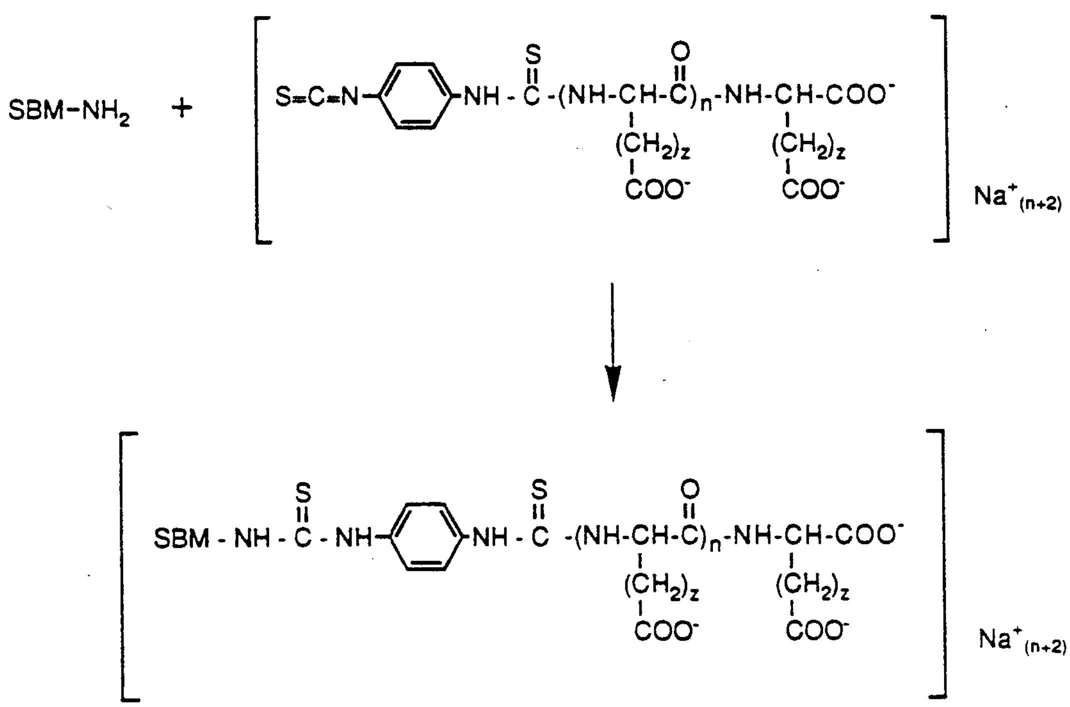

An "ancillary specific binding member", as used herein, refers to any member of a specific binding pair which is used in the assay in addition to the specific binding members of the capture reagent and the indicator reagent. For example, in an indirect assay an ancillary specific binding member may bind the analyte as well as a second specific binding member to which the analyte itself could not attach, or as in an inhibition assay the ancillary specific binding member may be a reference binding member as described below. One or more ancillary specific binding members can be used in an assay.

A "solid phase", as used herein, refers to any material which is insoluble, or can be made insoluble by a subsequent reaction. The solid phase can be chosen for its intrinsic charge and ability to attract the capture reagent, e.g., methylated wool, nylons, and special glasses having a positive charge. Alternatively, the solid phase can retain an additional charged substance that is oppositely charged with respect to the charged substance of the capture reagent. For example, an anionic substance can be bound to the capture reagent, and a cationic substance can be retained on the solid phase, or vice versa. Natural, synthetic, or naturally occurring materials that are synthetically modified, can be used as the cationic substance. A wide variety of proprietary polycations are available including hexadimethrine bromide (Polybrene®; Sigma Chemical Company, St. Louis, Mo), the GAFQuats (e.g., GafQuat™; GAF Corporation, Wayne, NJ, 07470), diethylaminoethyldextran (Sigma), and water soluble cellulose derivatives such as diallyldimethylammonium

chloride-hydroxyethyl cellulose polymer (Celquat™ L-200 and Celquat™ H-100, National Starch & Chemical Corporation, Bridgewater, NJ, 08807).

An assay device for the present invention can have many configurations, several of which are dependent upon the material chosen as the solid phase. For example, the solid phase can include any suitable porous material. By "porous" is meant that the material is one through which liquids can flow and can easily pass. In the present invention, the solid phase can include a fiberglass, cellulose, or nylon pad for use in a pour and flow-through assay device having one or more layers containing one or more of the assay reagents; a dipstick for a dip and read assay; a test strip for wicking (e.g., paper) or thin layer chromatographic (e.g., nitrocellulose) techniques; or other porous material well known to those skilled in the art. The solid phase, however, is not limited to porous materials. The solid phase can also comprise polymeric or glass beads, microparticles, tubes, sheets, plates, slides, wells, tapes, test tubes, or the like, or any other material which has an intrinsic charge or which can retain a charged substance.

Natural, synthetic, or naturally occurring materials that are synthetically modified, can be used as a solid phase including polysaccharides, e.g., cellulose materials such as paper and cellulose derivatives such as cellulose acetate and nitrocellulose; silica; inorganic materials such as deactivated alumina, diatomaceous earth, $MgSO_4$, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon); porous gels such as silica gel, agarose, dextran, and gelatin; polymeric films such as polyacrylamide; and the like. The solid phase should have reasonable strength or strength can be provided by means of a support, and it should not interfere with the production of a detectable signal.

Preferred solid phase materials include a porous fiberglass material, such as a "Whatman 934-AH" filter paper, which has a nominal thickness of 0.33 mm, or the disposable IMx™ wedge and TestPack™ (fiber matrix) devices of Abbott Laboratories (Abbott Park, IL, 60064). The thickness of such material is not critical, and will be a matter of choice, largely based upon the properties of the sample or analyte being assayed, such as the fluidity of the test sample.

To change or enhance the intrinsic charge of the solid phase, a charged substance can be coated directly to the material or onto microparticles which are then retained by a solid phase support material. Alternatively, microparticles alone can be used as the charged solid phase. Particles can serve as the solid phase, by being retained in a column or being suspended in the mixture of soluble reagents and test sample, or the particles themselves can be retained and immobilized by a solid phase support material. By "retained and immobilized" is meant that the particles on or in the support material are not capable of substantial movement to positions elsewhere within the support material. The particles can be selected by one skilled in the art from any suitable type of particulate material composed of polystyrene, polymethylacrylate, polypropylene, latex, polytetrafluoroethylene, polyacrylonitrile. polycarbonate, or similar materials. The size of the particles is not critical, although it is preferred that the average diameter of the particles be smaller than the average pore size of the support material being used.

Assays of the Invention

In accordance with the method of the present invention, a sandwich assay can be performed wherein a soluble capture reagent can include an analyte-specific binding member which has been bound to a charged substance such as an anionic substance. The ionic species can be a monomer or a polymer. The capture reagent is contacted with a test sample, suspected of containing the analyte, and an indicator reagent comprising a labeled analyte-specific binding member. The reagents can be mixed simultaneously or added sequentially, either singly or in combination. A binding reaction results in the formation of a capture reagent/analyte/indicator reagent complex. The assay also comprises the step of separating the resultant homogeneous complex from the excess reagents and test sample by using a solid phase that is either oppositely charged with respect to the capture reagent or that retains an oppositely charged substance, for example a cationic substance. In this example, the oppositely charged solid phase attracts and attaches to the capture reagent/analyte/indicator reagent complex through the interaction of the anionic and cationic substances. The complex retained on the solid phase is then detected by examining the solid phase for the indicator reagent. If analyte is present in the sample then label will be present on the solid phase material. The amount of label on the solid phase is proportional to the amount of analyte in the sample. The only major limitation inherent in the sandwich assay is the requirement for the analyte to have a sufficient size and appropriately orientated epitopes to permit binding of at least two specific binding members.

The present invention also can be used to conduct a competitive assay. In a competitive configuration, the soluble capture reagent again includes a specific binding member which has been attached to a charged substance, such as an anionic polymer. The capture reagent is contacted with both test sample and an indicator reagent that includes a second binding member which has been labeled with a signal generating compound. Either the capture reagent and analyte can compete in binding to the indicator reagent (e.g., the capture reagent and analyte are antigens competing for a labeled antibody), or the indicator reagent and analyte can compete in binding to the capture reagent (e.g., the indicator reagent is a labeled antigen which competes with the antigen analyte for binding to the antibody capture reagent). A competitive binding reaction occurs resulting in the formation of soluble complexes of (1) capture reagent/analyte or indicator reagent/analyte and (2) capture reagent/indicator reagent. The soluble complexes are removed from the excess reagents and test sample by contacting the reaction mixture with the oppositely charged solid phase, for example a cationic substance on a solid phase. The capture reagent complexes are retained on the solid phase through the interaction of the opposite charges. The complexes retained on the solid phase can be detected via the label of the indicator reagent. In the competitive assay, the amount of label that becomes associated with the solid phase is inversely proportional to the amount of analyte in the sample. Thus, a positive test sample will generate a negative signal. The competitive assay is advantageously used to determine the presence of small molecule analytes, such as small peptides or haptens, which have a single epitope with which to bind a specific binding partner. However, if the indicator reagent comprises a hapten conjugated to a label, then the competitive assay format wherein the indicator reagent and analyte compete in binding to the capture reagent is excluded.

The present invention can also be used in indirect immunoassays using one or more ancillary specific binding members. For example, an indirect sandwich immunoassay with the formation of a capture reagent/analyte/anti-analyte antibody/indicator reagent complex can be performed wherein the indicator reagent is a specific binding partner for the ancillary specific binding member which is specific for the analyte. In a further example, the capture reagent may include a specific binding partner for the ancillary specific binding member which is specific for the analyte.

In addition, the present invention can be used in an inhibition assay, such as the measurement of an antibody by inhibiting the detection of a reference antigen. For example, the capture reagent can include an antibody/anion conjugate and the indicator reagent can be a labeled-antibody. The test sample, suspected of containing an antibody analyte, is mixed with a reference antigen with which the capture reagent and indicator reagent can form a detectable sandwich complex that can be immobilized upon a solid phase. The degree of inhibition of antigen uptake by the capture reagent is proportional to the amount of antibody analyte in the test sample, thus, as the concentration of the antibody analyte increases, the less reference antigen is available to complete the immobilized sandwich complex.

In general, once complex formation occurs between the analyte and assay reagents the solid phase is used as a separation mechanism: the homogeneous reaction mixture is contacted with the solid phase, and the newly formed binding complexes are retained on the solid phase through the interaction of the opposite charges of the solid phase and the capture reagent. If the user is not concerned with liquid phase kinetics, the capture reagent can be preimmobilized on the solid phase to form a "capture situs", i.e., that region of the solid phase having one or more capture reagents non-diffusively attached thereto.

When the complex of charged capture reagent and analyte (and/or indicator reagent) is contacted to the oppositely charged solid phase, the ionic attraction of the oppositely charged species governs the efficiency of the separation of the complex from the reaction mixture. The ionic attraction can be selected to provide a greater attraction than the immunological attraction of antibody for antigen, particularly when multiple polycationic and polyanionic species are included in the capture reagent and solid phase. A further advantage is that the "ion-capture" technique minimizes the nonspecific adsorption of interfering substances onto the solid phase, thereby offering improved accuracy of analysis. The ion-capture technique thereby enables the performance of an assay having a highly specific separation method, minimal nonspecific binding, and high sensitivity.

## EXAMPLES

The following Examples illustrate preferred ways of making materials which can be used in the present invention and performing assay procedures using some of those materials. The Examples, however, are intended only to be illustrative, and are not to be construed as placing limitations upon the scope of the invention, which scope is defined solely by the appended claims.

**Example 1**

Activation of Poly-L-Glutamic Acid for the Formation of Anionic Capture Reagents

The following sequence of steps describes the chemistry used for the bulk preparation of protein-PGA conjugates for the formation of negatively charged capture reagents.

a. Conversion of PGA-sodium salt to the free acid form

The sodium salt of PGA (100 mg; 7.35 x $10^{-6}$ mole: average MW 13.600: Sigma) was stirred overnignt with a hydrogen form cation exchange resin (50 equivalents/glutamate residue; AG50W-X8; Bio-Rad). The resin previously had been swelled and washed in distilled water, and finally resuspended in distilled water (20 ml/7 gms dry weight of beads.) The supernatent was removed and lyophilized providing a 90% yield of the free acid form of PDA (PGAFA) as a white powder (80 mg: average MW 11,620). The free acid form was used to obtain solubility in organic solvents

b. Preparation of ITC-PGAFA

The PGAFA was dissolved in solvent (DMF at ten milligrams/milliliter.) A proton absorbing reagent (4-methyl morpholine) was added to the solution in the amount of about one equivalent per titratable free carboxylic acid. Next, about a 100 mole excess of an amine-reactive modification reagent (1,4-phenylene diisothiocyanate [DITC] in sufficient DMF to dissolve it) was added to the solution. The reaction mixture was stirred at room temperature overnight. The reaction mixture was rotavaporated to near dryness, and methylene chloride (25 ml) was added dropwise to precipitate the ITC-PGAFA. The flocculant precipitate was centrifuged, and the methylene chloride and unreacted DITC were removed. The precipitation/centrifugation process was repeated until substantially no detectable DITC remained. The DITC was detected using thin layer chromatography on silica slides developed in methylene chloride; ITC-PGAFA remains at the origin, DITC moves with the solvent front. The remaining solid was vacuum dried to yield the ITC-PGAFA as a yellow powder.

c. Coupling of ITC-PGAFA to protein to make capture reagents

The ITC-PGAFA (at a 1 to 20 mole excess to the protein) was dissolved in 0.2 M sodium phosphate buffer at pH 8.5 with the volume held as low as possible. The pH was adjusted to 8.5 as necessary. The desired protein was added to this solution and incubated overnight at 37°C. The preparations were then fractionated using HPLC on either an analytical TSK 400 Bio-Rad column (7.5 x 300 mm, at a 1 ml/min flow rate) for 1-2 milligram protein preparations, or a TSK 4000 Beckman column (31.5 x 300 mm, at a 5 ml/min flow rate) for 2-10 milligram protein preparations. The elution buffer contained 0.1 M sodium phosphate and 0.3 M NaCl at pH 6.8. Fractions were tested and appropriately combined. The amino acid content was determined for those fractions containing protein so that the coupling efficiency for the various proteins at various coupling ratios could be determined. The results of the determinations are presented in Table 1 .

Table 1

| Coupling Efficiencies of ITC-PGAFA with Various Proteins | | | |
|---|---|---|---|
| Protein | PGA Molar Excess | PGA Chain Number | Percent Substitution |
| Anti-CEA antibody | 1 | 0.77 | 77 |
| monoclonal 1.0 mg | 5 | 1.7 | 34 |
| | 10 | 3.1 | 31 |
| | 20 | 8.6 | 43 |
| Goat anti-rabbit antibody monoclonal 1.0 mg | 5 | 1.8 | 37 |
| Anti-βhCG antibody | 10 | 4.6 | 46 |
| monoclonal 1.0 mg | 15 | 5.2 | 36 |
| monoclonal 10 mg | 15 | 7.8 | 52 |
| Anti-digoxin antibody monoclonal 1.0 mg | 15 | 8.1 | 54 |
| monoclonal 5.0 mg | 15 | 5.5 | 37 |
| Goat anti-mouse antibody polyclonal 1.0 mg | 15 | 4.3 | 29 |
| Anti-T4 antibody monoclonal 1.0 mg | 15 | 6.9 | 46 |
| Anti-T4 antibody polyclonal 7.0 mg | 15 | 13.8 | 92 |
| Rabbit Serum Albumin loaded with Theophylline | 15 | 7.8 | 52 |

Column 1 of Table 1 lists the quantity of protein used in the reactions to form the various capture reagents. Column 2 lists the mole excess of activated ITC-PGAFA that was reacted with the Column 1 protein. Column 3 provides the number of PGA chains attached per antibody by the reaction, calculated by amino acid analysis based upon a 40,000 average MW and 305 repeating glutamate residues. Column 4 provides a calculation of the percent efficiency of PGA chain substitution based upon the mole excess of activated PGA used in the reaction.

Example 2

Theophylline Ion-Capture Competitive Assay-Antigen Capture Format

a. Preparation of theophylline capture reagent

The activation of theophylline was accomplished by dissolving theophylline-butylate (10 mg; MW 280.29; $3.57 \times 10^{-5}$ moles) in methylene chloride (3.0 ml). A three mole excess of dicyclohexylcarbodiimide (22 mg; MW 206.3) and a three mole excess of N-hydroxysuccinimide (12.3 mg; MW 115.09) were added, and the reaction mixture was stirred overnight at room temperature. The mixture was filtered to remove dicyclohexylurea and was rotavaporated to dryness to yield ten milligrams of N-succinimidyltheophylline-butylate (theophylline-butylate-oSu).

The free acid of polyglutamic acid ($NH_2$-PGAFA; 1.4 mg; MW 11,798; $1.19 \times 10^{-7}$ moles) was dissolved in DMF (0.5 ml) and NMM (1.1 mg; MW 101.15; $1.07 \times 10^{-5}$ moles) The theophylline-butylate-oSu (10 mg; at 1 mg/0.5 ml DMF) was added, and the reaction mixture was stirred overnight at room temperature. Unbound theophylline was removed by dialysis against a 0.1 M Na phosphate buffer at pH 7.0. The theophylline content of the resulting capture reagent was analyzed, and the results demonstrated that 3.9 theophylline molecules were attached per PGA chain. The theophyllin-PGA capture reagent, which was capable of binding with anti-theophylline antibody, was then diluted to 3 µg/ml in an assay buffer containing 25 mM Tris, 100 mM NaCl, 1 mM $MgCl_2$, 0.1 mM $ZnCl_2$, 0.1% $NaN_3$, and 1% fish gelatin at pH 7.2.

b. Preparation of the solid phase

A fiber matrix was coated with a polymeric quaternary compound to provide the solid phase with a positive charge. Celquat™ L-200, a water soluble cellulose derivative, was used. A 0.5% aqueous solution of Celquat™ L-200 (50 µl) containing 10 mM NaCl (50 µl) was applied to the solid phase material.

c. Preparation of the indicator reagent

The indicator reagent consisted of a conjugate of alkaline phosphatase and anti-theophylline antibody, prepared by coupling anti-Theophylline antibody to periodate activated alkaline phosphatase, in an assay buffer containing 25 mM Tris (hydroxymethyl) aminomethane, 100 mM NaCl, 1 mM $MgCl_2$, 0.1 mM $ZnCl_2$, 0,07% $NaN_3$, and 1% fish gelatin at pH 7.5.

The indicator reagent was appropriately diluted (as determined by titer curve) in the assay buffer to give 0.17 micrograms of antibody/milliliter.

d. Immunoassay protocol

The indicator reagent (200 $\mu$l) was placed within a series of reaction tubes. A theophylline standard solution (200 $\mu$l; theophylline-butylate diluted to 0.6, 1.2, 2.5. 4.9, 9.9, 99.2, and 992 $\mu$g/ml in 50 mM Tris, 300 mM NaCl and 0.1% $NaN_3$ at pH 7.2) was then added to each tube. The mixture was incubated ten minutes at 37°C. Capture reagent (200 $\mu$l) was added to each tube, and the reaction mixtures were incubated ten minutes at 37°C. An aliquot of each reaction mixture (200 $\mu$l) was applied to the quat-treated solid ohase material, followed by one wash with diluent (75 $\mu$l). An enzyme substrate (70 $\mu$l; 1.2 mM 4-methylumbelliferyl-phosphate in a solution of 100 mM AMP, 1.0 mM $MgCl_2$, 0.1% $NaN_3$, and 4.0 mM tetramisole at pH 10.3) was added at 32°C for reaction with the indicator reagent, and the resulting rate of fluorescence was measured. The results of the assay are shown in Table 2. The results demonstrate that as the theophylline test sample concentration increased there was a corresponding decrease in the formation of capture reagent/indicator reagent complex, and therefore, the amount of detectable label associated with the solid phase decreased.

Table 2

Theophylline Ion-Capture Competitive Assay-Antigen Capture Format

Capture reagent: Theophylline-PGA

Indicator reagent: alkaline phosphatase-labeled anti-theophylline antibody

| Theophylline (ng/ml) | Rate of fluorescence (counts/sec/sec) |
|---|---|
| 0 | 255 |
| 0.6 | 250 |
| 1.2 | 212 |
| 2.5 | 202 |
| 4.9 | 196 |
| 9.9 | 168 |
| 99.2 | 68 |
| 992 | 16 |

Example 3

Phenylcyclidine Ion-Capture Competitive Assay-Antigen Capture Format

a. Preparation of Phenylcyclidine Capture Reagent

4,Hydroxy-Phenylcyclidine (1.1 mg; MW 259.37; 4.24 x $10^{-6}$ moles) was dissolved in tetrahydrofuran (THF; 0.5 ml). One-half milliliter of 10% phosgene in benzene was added (130 mole excess.) The reaction was allowed to proceed at room temperature for 2.5 hours. The solvent was evaporated under a stream of

nitrogen to yield a residue of phenylcyclidine-4-chloroformate.

The phenylcyclidine-4-chloroformate (1.1 mg) was dissolved in THF (0.5 ml). To this was added $NH_2$-PGAFA (1.7 mg; MW 11,798; 1.19 x $10^{-7}$ moles) dissolved in 1-methyl-2-pyrrolidinone (0.5 ml). The reaction was carried out overnight at room temperature and then rotavaporated to dryness. The product was dissolved in 0.1 M sodium phosphate (1.5 ml, pH 7.0). The precipitate was filtered, and the cloudy aqueous filtrate was extracted with methylene chloride until clear. The phenylcyclidin-PGA capture reagent, which was capable of binding with anti-phenylcyclidine antibody, was then diluted to 5 µg/ml in an assay buffer as described in Example 2.

b. Preparation of the solid phase

The solid phase was prepared substantially in accordance with the method described in Example 2.

c. Preparation of the indicator reagent

The indicator reagent consisted of a conjugate of alkaline phosphatase and anti-phenylcyclidine antibody. The indicator reagent was diluted 1/250 in the assay buffer as described in Example 2.

d. Immunoassay protocol

The indicator reagent (140 µl) was mixed with a series of samples (50 µl each) containing known amounts of phenylcyclidine (0.0, 25, 60, 120, 250 and 500 ng/ml prepared in human urine), and the mixtures were incubated for ten minutes at 32°C. The phenylcyclidine-PGA capture reagent (100 µl) was added, and the reaction mixtures were incubated for ten minutes. An aliquot of each reaction mixture (200 µl) was applied to a solid phase material. The solid phase was then washed, two times. An enzyme substrate (70 µl; as described in Example 2)was added, and the resulting rate of fluorescence was measured. The results of the assay are shown in Table 3. The results demonstrate that as the phenylcyclidine test sample concentration increased there was a corresponding decrease in the formation of capture reagent/indicator reagent complex, and therefore, the amount of detectable label associated with the solid phase decreased.

Table 3

Phenylcyclidine Ion-Capture Competitive Assay-Antigen Capture Format

Capture reagent: Phenylcyclidine-PGA

Indicator reagent: alkaline phosphatase-labeled anti-phenylcyclidine antibody

| Phenylcyclidine (ng/ml) | Rate of fluorescence (counts/sec/sec) |
|---|---|
| 0 | 570 |
| 25 | 133 |
| 60 | 60 |
| 120 | 33 |
| 250 | 18 |
| 500 | 9 |

Example 4

Digoxin Ion-Capture Competitive Assay - Antigen Capture Format

a. Preparation of a digoxin-IgG-PGA capture reagent

The digoxin-IgG-PGA capture reagent was prepared substantially in accordance with the method described in Example 1 c., with the following procedural modifications. The ITC-PGA (5 mg; 1.25 x $10^{-7}$ mole; in 1.0 ml of 0.1 M sodium phosphate at pH 8.5) was added to a buffered solution of rabbit IgG-digoxin (1 mg; 6.25 x $10^{-9}$ mole; in 1.4493 ml of 0.1 M sodium phosphate and 0.3 M NaCl at pH 8.5) to form the capture reagent. The solution was stirred and incubated overnight at 37°C. The preparation was then fractionated using HPLC on a BioSil 400 (Bio-Rad 300 mm x 7.5 mm gel filtration column) and eluted at one milliliter/minute with 0.1 M sodium phosphate and 0.3 M NaCl at pH 6.8. The digoxin-IgG-PGA capture reagent, which was capable of binding with anti-digoxin antibody, was then diluted to 3 µg/ml in an assay buffer as described in Example 2)

b. Preparation of the solid phase

The solid phase was prepared substantially in accordance with the method described in Example 2.

c. Preparation of the indicator reagent

The indicator reagent consisted of a conjugate of alkaline phosphatase and mouse anti-digoxin antibody (Immuno-search; Emeryville, California 94608). The indicator reagent was diluted to 33.3 ng/ml in the assay buffer as described in Example 2.

d. Immunoassay protocol

The indicator reagent (200 µl) was mixed with a series of samples (200 µl) containing known amounts of digoxin (0.5, 1.0, 2.5, 5.0 and 50.0 ng/ml prepared in normal human serum). The mixtures were incubated for 15 minutes at 37°C. The digoxin-IgG-PGA capture reagent (200 µl) was added, and the reaction mixtures were incubated for 15 minutes. An aliquot of each reaction mixture (200 µl) was applied to the solid phase material, followed by a wash. An enzyme substrate (70 µl; as described in Example 2) was added, and the resulting rate of fluorescence was measured. The results of the assay are shown in Table 4. The results demonstrate that as the digoxin test sample concentration increased there was a corresponding decrease in the formation of capture reagent/indicator reagent complex, and therefore, the amount of detectable label associated with the solid phase decreased.

**Table 4**

**Digoxin Ion-Capture Competitive Assay-Antigen Capture Format**

**Capture reagent: Digoxin-IgG-PGA**

**Indicator reagent: alkaline phosphatase-labeled anti-digoxin antibody**

| Digoxin (ng/ml) | Rate of fluorescence (counts/sec/sec) |
|---|---|
| 0 | 115 |
| 0.5 | 101 |
| 1.0 | 91 |
| 2.5 | 74 |
| 5.0 | 60 |
| 50.0 | 14 |

It will be appreciated by one skilled in the art that the concepts of the present invention are equally applicable to any homogeneous binding assays (wherein the signal generating ability of the label is not altered during the binding reaction) by using oppositely charged solid phase materials and capture reagents. The embodiments described in detail herein are intended as examples rather than as limitations.

Thus, the description of the invention is not intended to limit the invention to the particular embodiments described, but it is intended to encompass all equivalents and subject matter within the scope of the invention as set forth in the following claims.

**Description for the following Contracting State : AT**

## BACKGROUND OF THE INVENTION

1. Field of the Invention

This invention relates generally to the field of binding assay devices and methods. In particular, the present invention relates to novel methods useful in the performance of homogeneous immunoassays.

2. Description of Related Art

Various analytical procedures and devices are commonly employed in assays to determine the presence and/or concentration of substances of interest or clinical significance which may be present in biological liquids or other materials, Such substances are commonly termed "analytes" and can include antibodies, antigens, drugs, hormones, etc.

Immunoassay techniques take advantage of the mechanisms of the immune systems of higher organisms, wherein antibodies are produced in response to the presence of antigens which are pathogenic or foreign to the organisms. These antibodies and antigens, i.e., immunoreactants, are capable of binding with one another, thereby creating a highly specific reaction mechanism which can be used *in vitro* to determine the presence or concentration of that particular antigen in a biological sample.

There are several known immunoassay methods using immunoreactants, wherein at least one of the immunoreactants is labeled with a detectable component so as to be analytically identifiable. For example, the "sandwich" or "two-site" technique may involve the formation of a ternary complex between an antigen and two antibodies. A convenient method of detecting complex formation in such a technique is to provide one labeled antibody and an unlabeled antibody bound to a solid phase support such that the complex can readily be isolated. In this example, the amount of labeled antibody associated with the solid phase is directly proportional to the amount of analyte in the test sample.

An alternative technique is the "competitive" assay. In one example of a competitive assay, the capture mechanism again may use an antibody attached to an insoluble solid phase, but a labeled analyte (rather than a labeled antibody) competes with the analyte present in the test sample for binding to the immobilized antibody. Similarly, an immobilized analyte can compete with the analyte of interest for a labeled antibody. In these competitive assays, the quantity of captured labeled reagent is inversely proportional to the amount of analyte present in the sample.

Despite their great utility, there are disadvantages with such assay methods. First, the heterogenous reaction mixture of soluble and insoluble reagents, and liquid test sample, can retard the kinetics of the reaction. In comparison to a liquid phase reaction wherein all reagents are soluble, i.e. a homogeneous reaction mixture, the heterogenous reaction mixture can require longer incubation periods for equilibrium to be reached in the reaction mixture between the insoluble solid phase system, the free analyte in the test sample, the soluble labeled reagent, and the newly formed insoluble complex. Second, conventional methods of attaching binding members to the solid phase material, such as adsorption of antibody to the solid phase, can produce a solid phase which will readily bind substances other than the analyte. This is referred to as nonspecific binding and can interfere with the detection of a positive result. Third, with conventional immobilization methods, separate batches of manufactured solid phase reagents can contain variable amounts of immobilized binding member.

Of interest as background art to the present invention is EP-A-0 245 926 disclosing methods of measuring antigens or antibodies in biological fluids relying on the separation of complexes by means of specific binding phenomena.

Likewise, EP-A- 0 268 296 discloses immunoassay methods relying on the separation of complexes by means of specific binding phenomena.

Of further interest is EP-A-0 230 768 which discloses a specific binding assay involving coagglutination of a non-magnetic particle, to which is bound a capture reagent for the analyte, and a solid phase comprising magnetic particles. The coagglutination occurs by means of ionic interaction of the particles. The assay medium is subjected to a magnetic field gradient to separate the coagglutinated particles from the medium. After the separation, the medium or the particles are examined for the presence or amount of a specific binding member, which is affected by the presence of analyte in the sample.

## SUMMARY OF THE INVENTION

The present invention provides a method for detecting an analyte in a test sample, comprising the steps of:

a) contacting the sample with

a soluble capture reagent, comprising a hapten conjugated to a first charged substance, and

an indicator reagent, comprising a specific binding member conjugated to a label capable of producing a detectable signal, thereby forming a reaction mixture,

wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,

b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and

c) detecting said label associated with said solid phase or said reaction mixture as an indication of the presence or amount of the analyte in the sample.

The present invention also provides a method for detecting an analyte in a test sample, comprising the steps of:

a) contacting the sample with

a soluble capture reagent, comprising a specific binding member conjugated to a first charged substance, and

an indicator reagent, comprising a hapten conjugated to a label capable of producing a detectable signal, thereby forming a reaction mixture,

wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,

b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and

c) detecting said label associated with said solid phase or said reaction mixture as an indication of the presence or amount of the analyte in the sample.

The present invention provides methods using capture reagents which facilitate the observation of a detectable signal by separating the analyte and/or indicator reagent from the other assay reagents or test sample. The capture reagents may comprise one or more anionic molecules attached to a specific binding member. A readily adapted anionic molecule is an activated polymeric anionic molecule having the formula:

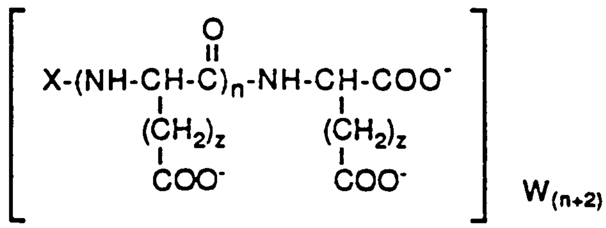

wherein n is about 10 to about 500; z is about 1 to about 6; W is chosen from $H^+$, $Na^+$, $K^+$, $Li^+$, amine salts such as $H^+NR_3$, and derivatives thereof; and X is an amine-reactive moiety, a thiol-reactive moiety or a thiol moiety with which the specific binding member will react. Alternatively, X can represent a specific binding member which has been activated to bind the polymeric anionic molecule. Activation methods are also described by which one or more reactive groups are formed upon the specific binding member or the polymeric anion.

The specific binding member component of the capture reagent can be either a napten or a macromolecule. The charged capture reagent enables homogeneous assay and separation reactions wherein the reaction complexes can be removed from the reaction mixture by contacting the mixture with an oppositely charged solid phase. Virtually any binding assay of the invention (sandwich assays, competitive assays, indirect assays using ancillary specific binding members, inhibition essays, etc.) can be carried out using the above capture reagents.

The present invention brings two advantages to binding assays: a) the use of liquid phase kinetics in the binding reaction facilitates the formation of a complex from the homogeneous mixture of analyte and assay reagents, and b) it increases the potential number of complexes that can be immobilized on the solid support.

If liquid phase kinetics are not sought, the present invention also provides an efficient method of immobilizing binding members on a solid phase through a method other than absorption, adsorption or covalent binding.

## DETAILED DESCRIPTION OF THE INVENTION

The assay methods of the present invention can be used in a variety of immunoassay formats. The present invention, however, is not limited to immunoreactive assays. Any assays using specific binding reactions between the analyte and assay reagents can be performed.

### Definitions

The following definitions are applicable to the present invention.

The term "specific binding member", as used herein, refers to a member of a specific binding pair, i.e., two different molecules where one of the molecules through chemical or physical means specifically binds to the second molecule. In addition to antigen and antibody-specific binding pairs, other specific binding pairs include biotin and avidin, carbohydrates and lectins, complementary nucleotide sequences (including probe and capture nucleic acid sequences used in DNA hybridization assays to detect a target nucleic acid sequence), complementary peptide sequences including those formed by recombinant methods, effector and receptor molecules, hormone and hormone binding protein, enzyme cofactors and enzymes, enzyme inhibitors and enzymes, and the like. Furthermore, specific binding pairs can include members that are analogs of the original specific binding member. For example, a derivative or fragment of the analyte, i.e., an analyte-analog, can be used so long as it has at least one epitope in common with the analyte. Immunoreactive specific binding members include antigens, haptens, antibodies, and complexes thereof including those formed by recombinant DNA methods or peptide synthesis. An antibody can be a monoclonal or polyclonal antibody, a recombinant protein or a mixture(s) or fragment(s) thereof, as well as a mixture of an antibody and other specific binding members. The details of the preparation of such antibodies and their suitability for use as specific binding members are well known to those skilled in the art.

The term "hapten", as used herein, refers to a partial antigen or non-protein binding member which is capable of binding to an antibody, but which is not capable of eliciting antibody formation unless coupled to a carrier protein.

The term "test sample", as used herein, refers to virtually any liquid sample. The test sample can be derived from any desired source, such as a physiological fluid, for example, blood, saliva, ocular lens fluid, cerebral spinal fluid, sweat, urine, milk, ascites fluid, mucous, synovial fluid, peritoneal fluid, amniotic fluid or the like. The liquid test sample can be pretreated prior to use, such as preparing plasma from blood, diluting viscous liquids, or the like; methods of treatment can also involve separation, filtration, distillation, concentration, inactivation of interfering components, and the addition of reagents. Besides physiological fluids, other liquid samples such as water, food products and the like can be used. In addition, a solid can be used once it is modified to form a liquid medium.

"Analyte", as used herein, is the substance to be detected in or separated from the test sample using the present invention. The analyte can be any substance for which there exists a naturally occurring specific binding member or for which a specific binding member can be prepared. In addition, the analyte may bind to more than one specific binding member. "Analyte" also includes any antigenic substances, haptens, antibodies, and combinations thereof. The analyte can include a protein, a peptide, an amino acid, a hormone, a steroid, a vitamin, a drug including those administered for therapeutic purposes as well as those administered for illicit purposes, a bacterium, a virus, and metabolites of or antibodies to any of the above substances.

The term "analyte-analog", as used herein, refers to a substance which cross-reacts with an analyte-specific binding member, although it may do so to a greater or a lesser extent than does the analyte itself. The analyte-analog can include a modified analyte as well as a fragmented or synthetic portion of the analyte molecule so long as the analyte-analog has at least one epitopic site in common with the analyte of interest.

The term "label", as used herein, refers to any substance which is attached to a specific binding member and which is capable of producing a signal that is detectable by visual or instrumental means. Various suitable labels for use in the present invention can include chromogens; catalysts; fluorescent compounds; chemiluminescent compounds; radioactive labels; direct visual labels including colloidal metallic and non-metallic particles, dye particles, enzymes or substrates, or organic polymer latex particles; liposomes or other vesicles containing signal producing substances; and the like.

A large number of enzymes suitable for use as labels are disclosed in U.S. Patent No. 4,275,149, columns 19-23. An example of an enzyme/substrate signal producing system useful in the present invention is the enzyme alkaline phosphatase and the substrate nitro blue tetrazolium-5-bromo-4-chloro-3-indolyl phosphate or a derivative or analog thereof.

In an alternative signal producing system, the label can be a fluorescent compound where no enzymatic manipulation of the label is required to produce a detectable signal. Fluorescent molecules such as fluorescein, phycobiliprotein, rhodamine and their derivatives and analogs are suitable for use as labels in this reaction.

In an especially preferred embodiment, a visually detectable, colored particle can be used as the label component of the indicator reagent, thereby providing for a direct colored readout of the presence or concentration of the analyte in the sample without the need for further signal producing reagents. Materials for use as the colored particles are colloidal metals, such as gold, and dye particles as disclosed in U.S. Pat. Nos. 4,313,734 and 4,373,932. The preparation and use of non-metallic colloids, such as colloidal selenium particles, are disclosed in EP-A-0 298 368 published on January 11, 1989 (co-owned and copending U.S. Patent Application Serial No. 072,084, filed July 9, 1987). The use of colloidal particle labels in immunochromatography is disclosed in EP-A-0 299 428 published on January 18, 1989 (co-owned and copending U.S. Patent Application Serial No. 072,459, filed July 13, 1987). Organic polymer latex particles for use as labels are disclosed in EP-A-0 360 088 published on March 28, 1990 (co-owned and copending U.S. Patent Application Serial No. 248,858, filed September 23, 1988).

A "signal producing component", as used herein, refers to any substance capable of reacting with another assay reagent or the analyte to produce a reaction product or signal that indicates the presence of the analyte and that is detectable by visual or instrumental means. "Signal production system", as used herein, refers to the group of assay reagents that are needed to produce the desired reaction product or signal. For example, one or more signal producing components can be used to react with a label and generate the detectable signal,i.e., when the label is an enzyme, amplification of the detectable signal is obtained by reacting the enzyme with one or more substrates or additional enzymes to produce a detectable reaction product.

An "indicator reagent", as used herein, refers to a label attached to a specific binding member. The indicator reagent produces a detectable signal at a level relative to the amount of an analyte in the test sample. Generally, the indicator reagent is detected or measured after it is captured on the solid phase material, but the unbound indicator reagent can also be measured to determine the result of an assay.

The specific binding member of the indicator reagent is capable of binding either to the analyte as in a sandwich assay, to the capture reagent as in a competitive assay, or to an ancillary specific binding member as in an indirect assay. The label, as described above, enables the indicator reagent to produce a detectable signal that is related to the amount of analyte in the test sample. The specific binding member component of the indicator reagent enables the indirect binding of the label to the analyte, to an ancillary specific binding member or to the capture reagent. The selection of a particular label is not critical, but the label will be capable of generating a detectable signal either by itself, such as a visually detectable signal generated by colored organic polymer latex particles, or in conjunction with one or more additional signal producing components, such as an enzyme/substrate signal producing system. A variety of different indicator reagents can be formed by varying either the label or the specific binding member; it will be appreciated by one skilled in the art that the choice involves consideration of the analyte to be detected and the desired means of detection.

A "capture reagent", as used herein, refers to an unlabeled specific binding member attached to a charged substance. The attachment of the components is essentially irreversible and can include covalent mechanisms. The capture reagent is used to facilitate the observation of the detectable signal by substantially separating the analyte and/or the indicator reagent from other assay reagents and the remaining test sample. The specific binding member can be a small molecule, such as a hapten or small peptide, so long as the attachment to the charged substance does not interfere with the binding member's binding site.

The specific binding member of the capture reagent is specific either for the analyte as in a sandwich assay, for the indicator reagent or analyte as in a competitive assay, or for an ancillary specific binding member, which itself is specific for the analyte, as in an indirect assay. The charged substance can include anionic and cationic monomers or polymers. For example, anionic polymers include polyglutamic acid (PGA), anionic protein or derivitized protein such as albumin, anionic polysaccharides such as heparin or alginic acid, polyaspartic acid, polyacrylic acid, and polyamino acids having a net negative charge at an appropriate pH (such as a pH in the range of 4 to 10.) Furthermore, the specific binding member can be joined to more than one charged monomer or polymer to increase the net charge associated with the capture reagent.

In one embodiment of the present invention, a negatively charged capture reagent can be prepared by conjugating a specific binding member to one or more activated polymeric anionic molecules and conjugate bases thereof represented by the general formula:

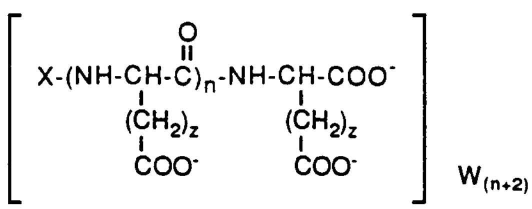

wherein n is 10 to 500; z is 1 to 6; W is chosen from $H^+$, $Na^+$, $K^+$, $Li^+$, amine salts such as $H^+NR_3$, and derivatives thereof; and X is virtually any reactive group or moiety having a reactive group that enables the chemical binding of the specific binding member and the polymer. X can be an amine-reactive group or moiety, a thiol-reactive group or moiety, or a thiol group or moiety represented by -A-SH wherein A is a spacer arm. For example, a specific binding member having an amino group can be conjugated to an activated PGA anionic molecule having an amine-reactive moiety. The amine-reactive moieties enable the binding of the activated polymer to an amino group on a specific binding member and include, but are not limited to, those represented by the following formulas

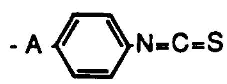 , 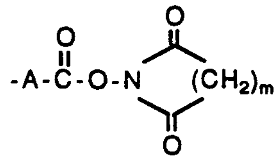 , 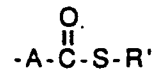 , 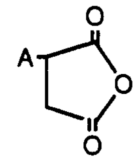 ,

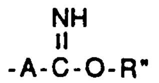 , 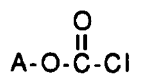 , 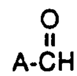 and the addition salts of 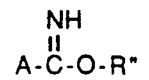 ,

wherein m is two or three, R' is a sulfur stabilizer and R" is an aliphatic or aryl group. Sulfur stabilizers include, but are not limited to, 2-pyridyl, 4-pyridyl and 5-nitro-2-pyridyl groups. "A" represents a spacer of one to thirty atoms including, but not limited to, carbon, nitrogen, sulfur and oxygen atom chains and combinations thereof such as polyether, polymethylene and polyamide, as well as aromatic spacers such as phenylthiocarbamyl.

Alternatively, a specific binding member having a thiol group can be conjugated to an activated polymer having a thiol-reactive moiety. The thiol-reactive moieties include, but are not limited to, those represented by the following formulas

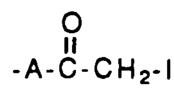 , 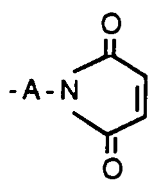 , 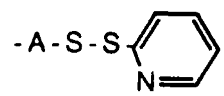 , 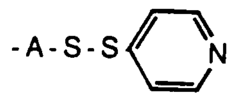 ,

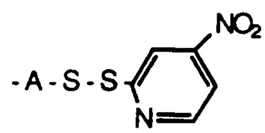 , 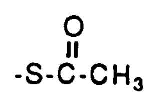 and 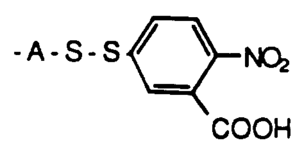

wherein A is a spacer of one to thirty atoms as described above. In yet another alternative, a specific binding member having a thiol-reactive group can be linked to an activated polymer having a thiol moiety such as -A-SH.

Typically, the negatively charged capture reagents of the following Examples were formed by reacting the desired specific binding member with an activated PGA molecule having modified terminal amino groups. Briefly, the modification method involved: 1) dissolving the PGA in a solvent (e.g., a water miscible aprotic solvent such as dioxane, dimethylformamide, 1-methyl-2-pyrrolidinone and dimethyl sulfoxide); 2) adding a proton absorbing reagent (e.g., 4-methyl morpholine)in the amount of about one equivalent per titratable carboxylic acid; 3) adding a 2 to a 100 molar excess of an amine-reactive modification reagent (e.g., 1,4-phenylene diisothiocyanate dissolved in dimethylformamide); 4) reacting the mixture; and 5) removing the unreacted amine-reactive modification reagent. Suitable proton absorbing reagents include alkali metal hydroxides such as sodium hydroxide, potassium hydroxide or lithium hydroxide, and tertiary amines such as 4-methyl morpholine and triethylamine.

The polymeric anionic molecule or the specific binding member will include one or more amino, carboxyl or thiol groups or can be activated by the incorporation of an amino, carboxyl or thiol group thereby enabling the chemical cross-linking of the specific binding member with the polymeric anionic molecule. "Activated species" refer to specific binding members and polymeric anionic molecules which contain a reactive group through the incorporation of a cross-linking or other activating agent. The amine-reactive modification reagents are a subclass of those reagents used to "activate" a specific binding member or polymeric anionic molecule, i.e., to prepare the specific binding member or the polymeric anionic molecule for chemical cross-linking. Activating agents also include thiol introducing agents such as the thiolanes (such as 2-iminothiolane), succinimidyl mercaptoacetates (such as N-succinimidyl-S-acetylmercaptoacetate), and disulfide compounds which are subsequently reduced to a thiol. The thiol introducing agents can be used to activate specific binding members and solid phase materials for their subsequent

reaction with a thiol-reactive group.

Amine-reactive modification reagents include, but are not limited to, bifunctional crosslinking or coupling agents, such as succinic anhydride analogs, iminothiolane analogs, homobifunctional reagents and heterobifunctional reagents, which enable the chemical cross-linking of the specific binding member and the polymeric anionic molecule. Examples of homobifunctional reagents can be represented by the formula X-A-X wherein X is an amine-reactive group and A is a spacer of one to thirty atoms. Examples of heterobifunctional reagents can be represented by the formula X-A-Y, wherein X is an amine-reactive group, Y is a thiol-reactive moiety, a thiol moiety or a thiol precursor and A is a spacer of one to thirty atoms as described above. Proteinaceous specific binding members with cysteine residues at the protein's active site can have their activity decreased by the addition of a coupling agent, therefore the cysteine residues in the active site must be protected, by means known in the art, prior to reacting the protein with the coupling agent.

The term "coupling agent", as used herein, includes bifunctional crosslinking or coupling agents, i.e., molecules containing two reactive groups or "ends", which may be tethered by a spacer. The reactive ends can be any of a variety of functionalities including, but not limited to: amino reacting ends such as N-hydroxysuccinimide (NHS) active esters, imidoesters, aldehydes, epoxides, sulfonyl halides, isocyanate, isothiocyanate, and nitroaryl halides; and thiol reacting ends such as pyridyl disulfides, maleimides, thiophthalimides, and active halogens. The heterobifunctional crosslinking reagents have two different reactive ends, e.g., an amino-reactive end and a thiol-reactive end, while homobifunctional reagents have two similar reactive ends, e.g., *bis*maleimidohexane (BMH) which permits the cross-linking of sulfhydryl-containing compounds, and NHS homobifunctional crosslinkers such as disuccinimidyl suberate (DSS) as well as the water soluble analogs, sulfo-NHS esters (Pierce 1989 Handbook and General Catalog; Pierce, Rockford, IL, 61105-9976).

Other commercially available homobifunctional cross-linking reagents include, but are not limited to, the imidoesters such as dimethyl adipimidate dihydrochloride (DMA); dimethyl pimelimidate dihydrochloride (DMP); and dimethyl suberimidate dihydrochloride (DMS). The iminothiolane analogs can be represented by the general formula:

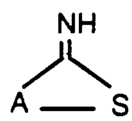

wherein A is a spacer of 1 to 5 atoms, e.g., 2-iminothiolane (Traut's Reagent.)

Commercially available heterobifunctional reagents suitable for use in the present invention include, but are not limited to, maleimido-NHS active esters coupling agents such as *m*-maleimidobenzoyl-N-hydroxysuccinimide ester (MBS); succinimidyl 4-(N-maleimidomethyl)cyclohexane-1-carboxylate (SMCC); succinimidyl 4-(*p*-maleimidophenyl)butyrate (SMPB) and derivatives thereof, including sulfosuccinimidyl derivatives such as sulfosuccinimidyl 4-(N-maleimido-methyl) cyclohexane-1-carboxylate (sulfo-SMCC); *m*-maleimidobenzoyl-sulfosuccinimide ester (sulfo-MBS) and sulfosucinimidyl 4-(*p*-maleimidophenyl)butyrate (sulfo-SMPB).

Other suitable heterobifunctional reagents include commercially available active halogen-NHS active esters coupling agents such as N-succinimidyl bromoacetate and N-succinimidyl(4-iodoacetyl)-aminobenzoate (SIAB) and the sulfosuccinimidyl derivatives such as sulfosuccinimidyl(4-iodoacetyl)-aminobenzoate (sulfo-SIAB). Another group of coupling agents is the heterobifunctional and thiol cleavable agents such as N-succinimidyl 3-(2-pyridyldithio)propionate (SPDP).

Yet another group of coupling agents includes the extended length heterobifunctional coupling agents described in EP-A-0 314 127 published on May 3, 1989 (co-owned and copending U.S. Patent Application Serial Number 114,930, filed October 30, 1987). The extended length heterobifunctional coupling agents include maleimido-NHS active ester reagents wherein the spacer is represented by the formula:

$$-\ (\text{amino acid})_n - \overset{O}{\overset{\|}{C}} - R -$$

wherein the amino acid is a substituted or unsubstituted amino acid, having from three to ten carbon atoms in a straight chain; n is from one to ten; and R is an alkyl, cycloalkyl, alkyl-cycloalkyl or an aromatic carboxylic ring. The term alkyl-cycloalkyl includes alkyl groups linked to cycloalkyl ring structures where the alkyl group links the cycloalkyl to a maleimide or carbonyl group. The term alkyl includes straight or branched alkyl groups, preferably lower alkyl groups having from one to six carbon atoms.

If a spacer is present, the spacer can be any molecular chain that is non-reactive, stable and non-binding to the analyte or other specific binding members with which it will be used. The length of the spacer can be varied and can range from the size of a single atom to the sizes disclosed in U.S. Patent Applications Serial Numbers 254,288 and 114,930 or larger.

The choice of the amine-reactive modification reagent, thiol introducing agent or other activating agent is not critical, but one skilled in the art will know of suitable or preferred agents for use with the particular polymeric anionic molecule and specific binding member to be used in the diagnostic assay. Therefore, it will be appreciated by those skilled in the art that the coupling agent or activating agent used in a given assay will generally be determined empirically.

Suitable thiol-reactive moieties of the heterobifunctional reagents include, but are not limited to, those represented by the following formulas:

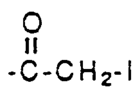 , 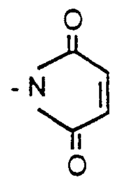 , 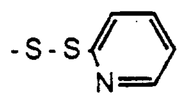 , 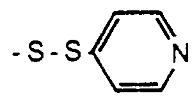 ,

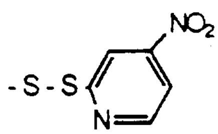 , and 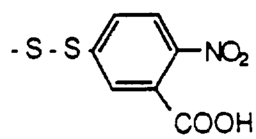

Suitable thiol precursor moieties include, but are not limited to, those represented by the following formulas:

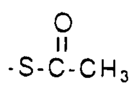 , 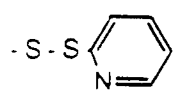 , 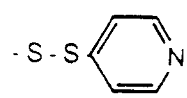 ,

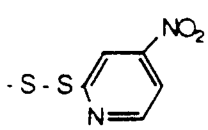 , and 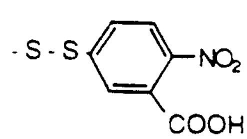

Suitable amine-reactive moieties include, but are not limited to, those represented by the following formulas:

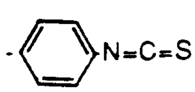 , 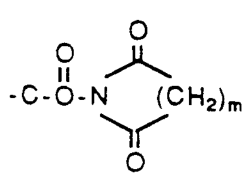 , 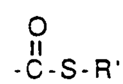 , 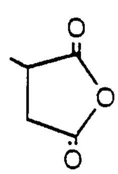 ,

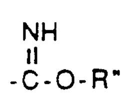 , 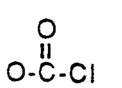 , 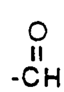 , and the addition salts of 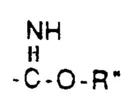 ,

wherein m is 2 or 3, R' is a sulfur stabilizer, as described above, and R" is an aliphatic or aryl group.

In yet another embodiment of the present invention, a specific binding member having an amine-reactive group (e.g., an activated specific binding member) can be conjugated to a terminal amino group of the polymeric anionic molecule. Briefly, an example of a conjugation procedure involves: 1) dissolving PGA in a solvent (e.g., a water miscible aprotic solvent such as dioxane, dimethylformamide, 1-methyl-2-pyrrolidinone and dimethyl sulfoxide); 2) adding a proton absorbing reagent (e.g., an alkali metal hydroxide such as sodium hydroxide, potassium hydroxide, or lithium hydroxide, or a tertiary amine such as 4-methyl morpholine or triethylamine).in the amount of about one equivalent per titratable carboxylic acid; 3) adding a 2 to a 100 molar excess of amine-reactive specific binding member (e.g., phosgen-activated phenylcyclidine or phenylcyclidine-4-chloroformate); 4) reacting the mixture and 5) removing the unreacted amine-reactive specific binding member. Suitable examples of amine-reactive groups on specific binding members include, but are not limited to,

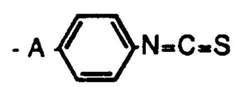 , 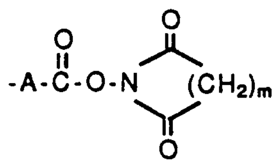 , 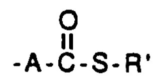 , 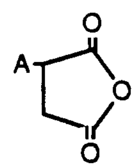 ,

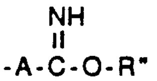 , 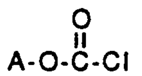 , 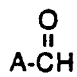 and the addition salts of 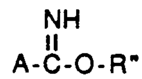 ,

wherein A is a spacer of about one to about thirty atoms as described above, m is two or three, R' is a sulfur stabilizer and R'' is an aliphatic or aryl group.

An example of the preparation of a negatively charged capture reagent involves the reaction of a specific binding member (SBM) having an amino group and an activated PGA having an amine-reactive moiety. The resulting reaction and reaction product can be illustrated as follows:

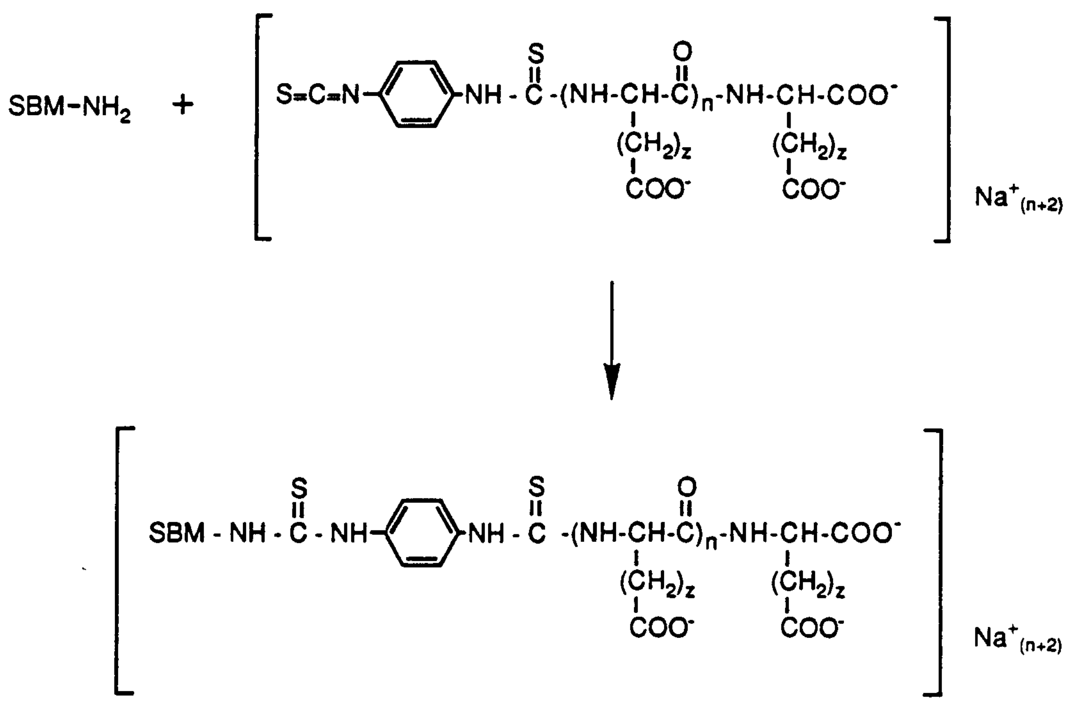

An "ancillary specific binding member", as used herein, refers to any member of a specific binding pair which is used in the assay in addition to the specific binding members of the capture reagent and the indicator reagent. For example, in an indirect assay an ancillary specific binding member may bind the analyte as well as a second specific binding member to which the analyte itself could not attach, or as in an inhibition assay the ancillary specific binding member may be a reference binding member as described below. One or more ancillary specific binding members can be used in an assay.

A "solid phase", as used herein, refers to any material which is insoluble, or can be made insoluble by a subsequent reaction. The solid phase can be chosen for its intrinsic charge and ability to attract the capture reagent, e.g., methylated wool, nylons, and special glasses having a positive charge. Alternatively, the solid phase can retain an additional charged substance that is oppositely charged with respect to the charged substance of the capture reagent. For example, an anionic substance can be bound to the capture reagent, and a cationic substance can be retained on the solid phase, or vice versa. Natural, synthetic, or naturally occurring materials that are synthetically modified, can be used as the cationic substance. A wide variety of proprietary polycations are available including hexadimethrine bromide (Polybrene®; Sigma Chemical Company, St. Louis, Mo), the GAFQuats (e.g., GafQuat™; GAF Corporation, Wayne, NJ, 07470), diethylaminoethyldextran (Sigma), and water soluble cellulose derivatives such as diallyldimethylammonium chloride-hydroxyethyl cellulose polymer (Celquat™ L-200 and Celquat™ H-100, National Starch & Chemical Corporation, Bridgewater, NJ, 08807).

An assay device for the present invention can have many configurations, several of which are dependent upon the material chosen as the solid phase. For example, the solid phase can include any suitable porous material. By "porous" is meant that the material is one through which liquids can flow and can easily pass. In the present invention, the solid phase can include a fiberglass, cellulose, or nylon pad for use in a pour and flow-through assay device having one or more layers containing one or more of the assay reagents; a dipstick for a dip and read assay; a test strip for wicking (e.g., paper) or thin layer chromatographic (e.g., nitrocellulose) techniques; or other porous material well known to those skilled in the art. The solid phase, however, is not limited to porous materials. The solid phase can also comprise polymeric or glass beads, microparticles, tubes, sheets, plates, slides, wells, tapes, test tubes, or the like, or any other material which has an intrinsic charge or which can retain a charged substance.

Natural, synthetic, or naturally occurring materials that are synthetically modified, can be used as a solid phase including polysaccharides, e.g., cellulose materials such as paper and cellulose derivatives such as cellulose acetate and nitrocellulose; silica; inorganic materials such as deactivated alumina, diatomaceous earth, $MgSO_4$, or other inorganic finely divided material uniformly dispersed in a porous polymer matrix, with polymers such as vinyl chloride, vinyl chloride-propylene copolymer, and vinyl chloride-vinyl acetate copolymer; cloth, both naturally occurring (e.g., cotton) and synthetic (e.g., nylon); porous gels such as silica gel, agarose, dextran, and gelatin; polymeric films such as polyacrylamide; and the like. The solid phase should have reasonable strength or strength can be provided by means of a support, and it should not interfere with the production of a detectable signal.

Preferred solid phase materials include a porous fiberglass material, such as a "Whatman 934-AH" filter paper, which has a nominal thickness of 0.33 mm, or the disposable IMx™ wedge and TestPack™ (fiber matrix) devices of Abbott Laboratories (Abbott Park, IL, 60064). The thickness of such material is not critical, and will be a matter of choice, largely based upon the properties of the sample or analyte being assayed, such as the fluidity of the test sample.

To change or enhance the intrinsic charge of the solid phase, a charged substance can be coated directly to the material or onto microparticles which are then retained by a solid phase support material. Alternatively, microparticles alone can be used as the charged solid phase. Particles can serve as the solid phase, by being retained in a column or being suspended in the mixture of soluble reagents and test sample, or the particles themselves can be retained and immobilized by a solid phase support material. By "retained and immobilized" is meant that the particles on or in the support material are not capable of substantial movement to positions elsewhere within the support material. The particles can be selected by one skilled in the art from any suitable type of particulate material composed of polystyrene, polymethylacrylate, polypropylene, latex, polytetrafluoroethylene, polyacrylonitrile polycarbonate, or similar materials. The size of the particles is not critical, although it is preferred that the average diameter of the particles be smaller than the average pore size of the support material being used.

Assays of the Invention

In accordance with the method of the present invention, a sandwich assay can be performed wherein a soluble capture reagent can include an analyte-specific binding member which has been bound to a charged substance such as an anionic substance. The ionic species can be a monomer or a polymer. The capture reagent is contacted with a test sample, suspected of containing the analyte, and an indicator reagent comprising a labeled analyte-specific binding member. The reagents can be mixed simultaneously or added sequentially, either singly or in combination. A binding reaction results in the formation of a capture reagent/analyte/indicator reagent complex. The assay also comprises the step of separating the resultant homogeneous complex from the excess reagents and test sample by using a solid phase that is

either oppositely charged with respect to the capture reagent or that retains an oppositely charged substance, for example a cationic substance. In this example, the oppositely charged solid phase attracts and attaches to the capture reagent/analyte/indicator reagent complex through the interaction of the anionic and cationic substances. The complex retained on the solid phase is then detected by examining the solid phase for the indicator reagent. If analyte is present in the sample, then label will be present on the solid phase material. The amount of label on the solid phase is proportional to the amount of analyte in the sample. The only major limitation inherent in the sandwich assay is the requirement for the analyte to have a sufficient size and appropriately orientated epitopes to permit binding of at least two specific binding members.

The present invention also can be used to conduct a competitive assay. In a competitive configuration, the soluble capture reagent again includes a specific binding member which has been attached to a charged substance, such as an anionic polymer. The capture reagent is contacted with both test sample and an indicator reagent that includes a second binding member which has been labeled with a signal generating compound. Either the capture reagent and analyte can compete in binding to the indicator reagent (e.g., the capture reagent and analyte are antigens competing for a labeled antibody), or the indicator reagent and analyte can compete in binding to the capture reagent (e.g., the indicator reagent is a labeled antigen which competes with the antigen analyte for binding to the antibody capture reagent). A competitive binding reaction occurs resulting in the formation of soluble complexes of (1) capture reagent/analyte or indicator reagent/analyte and (2) capture reagent/indicator reagent. The soluble complexes are removed from the excess reagents and test sample by contacting the reaction mixture with the oppositely charged solid phase, for example a cationic substance on a solid phase. The capture reagent complexes are retained on the solid phase through the interaction of the opposite charges. The complexes retained on the solid phase can be detected via the label of the indicator reagent. In the competitive assay, the amount of label that becomes associated with the solid phase is inversely proportional to the amount of analyte in the sample. Thus, a positive test sample will generate a negative signal. The competitive assay is advantageously used to determine the presence of small molecule analytes, such as small peptides or haptens, which have a single epitope with which to bind a specific binding partner.

For example, in an assay for theophylline, an anti-theophylline antibody (either monoclonal or polyclonal) can be conjugated with an anionic substance to form a soluble capture reagent, and a competition for binding to that antibody can be established between the soluble labeled theophylline (i.e., indicator reagent) and the unlabeled theophylline of the test sample. After incubation, the homogeneous mixture can be contacted to a cation-coated solid phase. The attraction between the oppositely charged ionic species of the capture reagent and the solid phase separates the immunocomplex from the reaction mixture. The signal from the indicator reagent can then be detected. In this example, increased theophylline levels in the test sample will result in decreased signal generation associated with the solid phase.

The present invention can also be used in indirect immunoassays using one or more ancillary specific binding members. For example, an indirect sandwich immunoassay with the formation of a capture reagent/analyte/anti-analyte antibody/indicator reagent complex can be performed, wherein the indicator reagent is a specific binding partner for the ancillary specific binding member which is specific for the analyte. In a further example, the capture reagent may include a specific binding partner for the ancillary specific binding member which is specific for the analyte.

In addition, the present invention can be used in an inhibition assay, such as the measurement of an antibody by inhibiting the detection of a reference antigen. For example, the capture reagent can include an antibody/anion conjugate and the indicator reagent can be a labeled-antibody. The test sample, suspected of containing an antibody analyte, is mixed with a reference antigen with which the capture reagent and indicator reagent can form a detectable sandwich complex that can be immobilized upon a solid phase. The degree of inhibition of antigen uptake by the capture reagent is proportional to the amount of antibody analyte in the test sample, thus, as the concentration of the antibody analyte increases, the less reference antigen is available to complete the immobilized sandwich complex.

In general, once complex formation occurs between the analyte and assay reagents, the solid phase is used as a separation mechanism: the homogeneous reaction mixture is contacted with the solid phase, and the newly formed binding complexes are retained on the solid phase through the interaction of the opposite charges of the solid phase and the capture reagent. If the user is not concerned with liquid phase kinetics, the capture reagent can be pre-immobilized on the solid phase to form a "capture situs", i.e., that region of the solid phase having one or more capture reagents non-diffusively attached thereto.

When the complex of charged capture reagent and analyte (and/or indicator reagent) is contacted to the oppositely charged solid phase, the ionic attraction of the oppositely charged species governs the efficiency of the separation of the complex from the reaction mixture. The ionic attraction can be selected to provide a

greater attraction than the immunological attraction of antibody for antigen, particularly when multiple polycationic and polyanionic species are included in the capture reagent and solid phase. A further advantage is that the "ion-capture" technique minimizes the nonspecific adsorption of interfering substances onto the solid phase, thereby offering improved accuracy of analysis. The ion-capture technique thereby enables the performance of an assay having a highly specific separation method, minimal nonspecific binding, and high sensitivity.

## EXAMPLES

The following Examples illustrate preferred ways of making materials which can be used in the present invention and performing assay procedures using some of those materials. The Examples, however, are intended only to be illustrative, and are not to be construed as placing limitations upon the scope of the invention, which scope is defined solely by the appended claims.

### Example 1

Activation of Poly-L-Glutamic Acid for the Formation of Anionic Capture Reagents

The following sequence of steps describes the chemistry used for the bulk preparation of protein-PGA conjugates for the formation of negatively charged capture reagents.

a. Conversion of PGA-sodium salt to the free acid form

The sodium salt of PGA (100 mg: 7.35 x $10^{-6}$ mole: average MW 13.600: Sigma) was stirred overnight with a hydrogen form cation exchange resin (50 equivalents/glutamate residue; AG50W-X8: Bio-Rad). The resin previously had been swelled and washed in distilled water, and finally resuspended in distilled water (20 ml/7 gms dry weight of beads.) The supernatent was removed and lyophilized providing a 90% yield of the free acid form of PGA (PGAFA) as a white powder (80 mg; average MW 11,620). The free acid form was used to obtain solubility in organic solvents

b. Preparation of ITC-PGAFA

The PGAFA was dissolved in solvent (DMF at ten milligrams/milliliter.) A proton absorbing reagent (4-methyl morpholine) was added to the solution in the amount of about one equivalent per titratable free carboxylic acid. Next, about a 100 mole excess of an amine-reactive modification reagent (1,4-phenylene diisothiocyanate [DITC] in sufficient DMF to dissolve it) was added to the solution. The reaction mixture was stirred at room temperature overnight. The reaction mixture was rotavaporated to near dryness, and methylene chloride (25 ml) was added dropwise to precipitate the ITC-PGAFA. The flocculant precipitate was centrifuged, and the methylene chloride and unreacted DITC were removed. The precipitation/centrifugation process was repeated until substantially no detectable DITC remained. The DITC was detected using thin layer chromatography on silica slides developed in methylene chloride; ITC-PGAFA remains at the origin. DITC moves with the solvent front. The remaining solid was vacuum dried to yield the ITC-PGAFA as a yellow powder.

c. Coupling of ITC-PGAFA to protein to make capture reagents

The ITC-PGAFA (at a 1 to 20 mole excess to the protein) was dissolved in 0.2 M sodium phosphate buffer at pH 8.5 with the volume held as low as possible. The pH was adjusted to 8.5 as necessary. The desired protein was added to this solution and incubated overnight at 37 °C. The preparations were then fractionated using HPLC on either an analytical TSK 400 Bio-Rad column (7.5 x 300 mm, at a 1 ml/min flow rate) for 1-2 milligram protein preparations, or a TSK 4000 Beckman column (31.5 x 300 mm, at a 5 ml/min flow rate) for 2-10 milligram protein preparations. The elution buffer contained 0.1 M sodium phosphate and 0.3 M NaCl at pH 6.8. Fractions were tested and appropriately combined. The amino acid content was determined for those fractions containing protein so that the coupling efficiency for the various proteins at various coupling ratios could be determined. The results of the determinations are presented in Table 1 .

Table 1

| Coupling Efficiencies of ITC-PGAFA with Various Proteins | | | |
|---|---|---|---|
| Protein | PGA Molar Excess | PGA Chain Number | Percent Substitution |
| Anti-CEA antibody | 1 | 0.77 | 77 |
| monoclonal 1.0 mg | 5 | 1.7 | 34 |
| | 10 | 3.1 | 31 |
| | 20 | 8.6 | 43 |
| Goat anti-rabbit antibody monoclonal 1.0 mg | 5 | 1.8 | 37 |
| Anti-βhCG antibody | 10 | 4.6 | 46 |
| monoclonal 1.0 mg | 15 | 5.2 | 36 |
| monoclonal 10 mg | 15 | 7.8 | 52 |
| Anti-digoxin antibody monoclonal 1.0 mg | 15 | 8.1 | 54 |
| monoclonal 5.0 mg | 15 | 5.5 | 37 |
| Goat anti-mouse antibody polyclonal 1.0 mg | 15 | 4.3 | 29 |
| Anti-T4 antibody monoclonal 1.0 mg | 15 | 6.9 | 46 |
| Anti-T4 antibody polyclonal 7.0 mg | 15 | 13.8 | 92 |
| Rabbit Serum Albumin loaded with Theophylline | 15 | 7.8 | 52 |

Column 1 of Table 1 lists the quantity of protein used in the reactions to form the various capture reagents. Column 2 lists the mole excess of activated ITC-PGAFA that was reacted with the Column 1 protein. Column 3 provides the number of PGA chains attached per antibody by the reaction, calculated by amino acid analysis based upon a 40,000 average MW and 305 repeating glutamate residues. Column 4 provides a calculation of the percent efficiency of PGA chain substitution based upon the mole excess of activated PGA used in the reaction.

Example 2

Theophylline Ion-Capture Competitive Assay-Antigen Capture Format

a. Preparation of theophylline capture reagent

The activation of theophylline was accomplished by dissolving theophylline-butylate (10 mg; MW 280.29; $3.57 \times 10^{-5}$ moles) in methylene chloride (3.0 ml). A three mole excess of dicyclohexylcarbodiimide (22 mg; MW 206.3) and a three mole excess of N-hydroxysuccinimide (12.3 mg; MW 115.09) were added, and the reaction mixture was stirred overnight at room temperature. The mixture was filtered to remove dicyclohexylurea and was rotavaporated to dryness to yield ten milligrams of N-succinimidyltheophylline-butylate (theophylline-butylate-oSu).

The free acid of polyglutamic acid ($NH_2$-PGAFA; 1.4 mg; MW 11,798; $1.19 \times 10^{-7}$ moles) was dissolved in DMF (0.5 ml) and NMM (1.1 mg; MW 101.15; $1.07 \times 10^{-5}$ moles) The theophylline-butylate-oSu (10 mg; at 1 mg/0.5 ml DMF) was added, and the reaction mixture was stirred overnight at room temperature. Unbound theophylline was removed by dialysis against a 0.1 M Na phosphate buffer at pH 7.0. The theophylline content of the resulting capture reagent was analyzed, and the results demonstrated that 3.9 theophylline molecules were attached per PGA chain. The theophylline-PGA capture reagent, which was capable of binding with anti-theophylline antibody, was then diluted to 3 µg/ml in an assay buffer containing 25 mM Tris, 100 mM NaCl, 1 mM $MgCl_2$, 0.1 mM $ZnCl_2$, 0.1% $NaN_3$, and 1% fish gelatin at pH 7.2.

b. Preparation of the solid phase

A fiber matrix was coated with a polymeric quaternary compound to provide the solid phase with a positive charge. Celquat™ L-200, a water soluble cellulose derivative, was used. A 0.5% aqueous solution of Celquat™ L-200 (50 µl) containing 10 mM NaCl (50 µl) was applied to the solid phase material.

c. Preparation of the indicator reagent

The indicator reagent consisted of a conjugate of alkaline phosphatase and anti-theophylline antibody, prepared by coupling anti-Theophylline antibody to periodate activated alkaline phosphatase, in an assay buffer containing 25 mM Tris (hydroxymethyl) aminomethane, 100 mM NaCl, 1 mM $MgCl_2$, 0.1 mM $ZnCl_2$, 0,07% $NaN_3$, and 1% fish gelatin at pH 7.5.

The indicator reagent was appropriately diluted (as determined by titer curve) in the assay buffer to give 0.17 micrograms of antibody/milliliter.

d. Immunoassay protocol

The indicator reagent (200 µl) was placed within a series of reaction tubes. A theophylline standard solution (200 µl; theophylline-butylate diluted to 0.6, 1.2, 2.5, 4.9, 9.9, 99.2, and 992 µg/ml in 50 mM Tris, 300 mM NaCl and 0.1% $NaN_3$ at pH 7.2) was then added to each tube. The mixture was incubated ten minutes at 37°C. Capture reagent (200 µl) was added to each tube, and the reaction mixtures were incubated ten minutes at 37°C. An aliquot of each reaction mixture (200 µl) was applied to the quat-treated solid phase material, followed by one wash with diluent (75 µl). An enzyme substrate (70 µl; 1.2 mM 4-methylumbelliferyl-phosphate in a solution of 100 mM AMP, 1.0 mM $MgCl_2$, 0.1% $NaN_3$, and 4.0 mM tetramisole at pH 10.3) was added at 32°C for reaction with the indicator reagent, and the resulting rate of fluorescence was measured. The results of the assay are shown in Table 2. The results demonstrate that as the theophylline test sample concentration increased there was a corresponding decrease in the formation of capture reagent/indicator reagent complex, and therefore, the amount of detectable label associated with the solid phase decreased.

Table 2

Theophylline Ion-Capture Competitive Assay-Antigen Capture Format

Capture reagent: Theophylline-PGA

Indicator reagent: alkaline phosphatase-labeled anti-theophylline antibody

| Theophylline (ng/ml) | Rate of fluorescence (counts/sec/sec) |
|---|---|
| 0 | 255 |
| 0.6 | 250 |
| 1.2 | 212 |
| 2.5 | 202 |
| 4.9 | 196 |
| 9.9 | 168 |
| 99.2 | 68 |
| 992 | 16 |

Example 3

Phenylcyclidine Ion-Capture Competitive Assay-Antigen Capture Format

a. Preparation of Phenylcyclidine Capture Reagent

4,Hydroxy-Phenylcyclidine (1.1 mg; MW 259.37; 4.24 x $10^{-6}$ moles) was dissolved in tetrahydrofuran (THF; 0.5 ml). One-half milliliter of 10% phosgene in benzene was added (130 mole excess.) The reaction

was allowed to proceed at room temperature for 2.5 hours. The solvent was evaporated under a stream of nitrogen to yield a residue of phenylcyclidine-4-chloroformate.

The phenylcyclidine-4-chloroformate (1.1 mg) was dissolved in THF (0.5 ml). To this was added $NH_2$-PGAFA (1.7 mg; MW 11,798; 1.19 x $10^{-7}$ moles) dissolved in 1-methyl-2-pyrrolidinone (0.5 ml). The reaction was carried out overnight at room temperature and then rotavaporated to dryness. The product was dissolved in 0.1 M sodium phosphate (1.5 ml, pH 7.0). The precipitate was filtered, and the cloudy aqueous filtrate was extracted with methylene chloride until clear. The phenylcyclidine-PGA capture reagent, which was capable of binding with anti-phenylcyclidine antibody, was then diluted to 5 µg/ml in an assay buffer as described in Example 2.

b. Preparation of the solid phase

The solid phase was prepared substantially in accordance with the method described in Example 2.

c. Preparation of the indicator reagent

The indicator reagent consisted of a conjugate of alkaline phosphatase and anti-phenylcyclidine antibody. The indicator reagent was diluted 1/250 in the assay buffer as described in Example 2.

d. Immunoassay protocol

The indicator reagent (140 µl) was mixed with a series of samples (50 µl each) containing known amounts of phenylcyclidine (0.0, 25, 60, 120, 250 and 500 ng/ml prepared in human urine), and the mixtures were incubated for ten minutes at 32 °C. The phenylcyclidine-PGA capture reagent (100 µl) was added, and the reaction mixtures were incubated for ten minutes. An aliquot of each reaction mixture (200 µl) was applied to a solid phase material. The solid phase was then washed, two times. An enzyme substrate (70 µl; as described in Example 2)was added, and the resulting rate of fluorescence was measured. The results of the assay are shown in Table 3. The results demonstrate that as the phenylcyclidine test sample concentration increased there was a corresponding decrease in the formation of capture reagent/indicator reagent complex, and therefore, the amount of detectable label associated with the solid phase decreased.

**Table 3**

**Phenylcyclidine Ion-Capture Competitive Assay-Antigen Capture Format**

**Capture reagent: Phenylcyclidine-PGA**

**Indicator reagent: alkaline phosphatase-labeled anti-phenylcyclidine antibody**

| Phenylcyclidine (ng/ml) | Rate of fluorescence (counts/sec/sec) |
|---|---|
| 0 | 570 |
| 25 | 133 |
| 60 | 60 |
| 120 | 33 |
| 250 | 18 |
| 500 | 9 |

Example 4

Digoxin Ion-Capture Competitive Assay - Antigen Capture Format

a. Preparation of a digoxin-IgG-PGA capture reagent

The digoxin-IgG-PGA capture reagent was prepared substantially in accordance with the method described in Example 1 c., with the following procedural modifications. The ITC-PGA (5 mg; 1.25 x $10^{-7}$ mole; in 1.0 ml of 0.1 M sodium phosphate at pH 8.5) was added to a buffered solution of rabbit IgG-digoxin (1 mg; 6.25 x $10^{-9}$ mole; in 1.4493 ml of 0.1 M sodium phosphate and 0.3 M NaCl at pH 8.5) to form the capture reagent. The solution was stirred and incubated overnight at 37°C. The preparation was then fractionated using HPLC on a BioSil 400 (Bio-Rad 300 mm x 7.5 mm gel filtration column) and eluted at one milliliter/minute with 0.1 M sodium phosphate and 0.3 M NaCl at pH 6.8. The digoxin-IgG-PGA capture reagent, which was capable of binding with anti-digoxin antibody, was then diluted to 3 µg/ml in an assay buffer as described in Example 2)

b. Preparation of the solid phase

The solid phase was prepared substantially in accordance with the method described in Example 2.

c. Preparation of the indicator reagent

The indicator reagent consisted of a conjugate of alkaline phosphatase and mouse anti-digoxin antibody (Immuno-search; Emeryville, California 94608). The indicator reagent was diluted to 33.3 ng/ml in the assay buffer as described in Example 2.

d. Immunoassay protocol

The indicator reagent (200 µl) was mixed with a series of samples (200 µl) containing known amounts of digoxin (0.5, 1.0, 2.5, 5.0 and 50.0 ng/ml prepared in normal human serum). The mixtures were incubated for 15 minutes at 37°C. The digoxin-IgG-PGA capture reagent (200 µl) was added, and the reaction mixtures were incubated for 15 minutes. An aliquot of each reaction mixture (200 µl) was applied to the solid phase material, followed by a wash. An enzyme substrate (70 µl; as described in Example 2) was added, and the resulting rate of fluorescence was measured. The results of the assay are shown in Table 4. The results demonstrate that as the digoxin test sample concentration increased there was a corresponding decrease in the formation of capture reagent/indicator reagent complex, and therefore, the amount of detectable label associated with the solid phase decreased.

Table 4

Digoxin Ion-Capture Competitive Assay-Antigen Capture Format

Capture reagent: Digoxin-IgG-PGA

Indicator reagent: alkaline phosphatase-labeled anti-digoxin antibody

| Digoxin (ng/ml) | Rate of fluorescence (counts/sec/sec) |
|---|---|
| 0 | 115 |
| 0.5 | 101 |
| 1.0 | 91 |
| 2.5 | 74 |
| 5.0 | 60 |
| 50.0 | 14 |

Example 5

Digoxin Ion-Capture Competitive Assay - Antibody Capture Format

a. Preparation of the indicator reagent

The indicator reagent consisted of a conjugate of alkaline phosphatase and digoxin (Immuno-search). The indicator reagent was diluted to 1/100 in the assay buffer as described in Example 2.

b. Immunoassay protocol

The anti-digoxin-PGA capture reagent (200 µl, prepared substantially in accordance with the protocol described in Example 1.c) was mixed with a series of samples (200 µl each) containing known amounts of digoxin as described in Example 4 . The mixtures were incubated for 15 minutes at 37°C. The indicator reagent (200 µl) was added, and the reaction mixtures were incubated for 15 minutes. An aliquot of each reaction mixture (200 µl) was applied to the solid phase (prepared as described in Example 2,) followed by a wash. An enzyme substrate (70 µl; as described in Example 2) was added, and the resulting rate of fluorescence was measured. The results of the assay are shown in Table 5. The results demonstrate that as the digoxin test sample concentration increased there was a corresponding decrease in the formation of capture reagent/indicator reagent complex, and therefore, the amount of detectable label associated with the solid phase decreased.

Table 5

Digoxin Ion-Capture Competitive Assay-Antibody Capture Format

Capture reagent: anti-digoxin antibody-PGA

Indicator reagent: alkaline phosphatase-labeled digoxin

| Digoxin (ng/ml) | Rate of fluorescence (counts/sec/sec) |
|---|---|
| 0 | 85 |
| 0.5 | 68 |
| 1.0 | 48 |
| 2.5 | 23 |
| 5.0 | 10 |
| 50.0 | 1 |

It will be appreciated by one skilled in the art that the concepts of the present invention are equally applicable to any homogeneous binding assays (wherein the signal generating ability of the label is not altered during the binding reaction) by using oppositely charged solid phase materials and capture reagents. The embodiments described in detail herein are intended as examples rather than as limitations.

Thus, the description of the invention is not intended to limit the invention to the particular embodiments described, but it is intended to encompass all equivalents and subject matter within the scope of the invention as set forth in the following claims.

**Claims**

**Claims for the following Contracting States : BE, CH, DE, ES, FR, GB, IT, LI, NL**

**1.** A method for detecting an analyte in a test sample, comprising the steps of:

a) contacting the sample with

a soluble capture reagent, comprising a hapten conjugated to a first charged substance, and

an indicator reagent, comprising a specific binding member conjugated to a label capable of

producing a detectable signal, thereby forming a reaction mixture,

wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,

b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and

c) detecting said label associated with said solid phase or said reaction mixture as an indication of the presence or amount of the analyte in the sample.

**2.** A method for detecting an analyte in a test sample, comprising the steps of:

a) contacting the sample with

a soluble capture reagent, comprising a specific binding member conjugated to a first charged substance, and

an indicator reagent, comprising a hapten conjugated to a label capable of producing a detectable signal, thereby forming a reaction mixture,

wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,

b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and

c) detecting said label associated with said solid phase or said reaction mixture as an indication of the presence or amount of the analyte in the sample,

provided that competitive assays wherein the indicator reagent and analyte compete in binding to the capture reagent are excluded.

**3.** The method according to Claim 1 or 2, wherein a second charged substance having an opposite charge with respect to said first charged substance is retained on said solid phase material.

**4.** The method according to Claim 3, wherein said first charged substance is a polymeric anionic substance and said second charged substance is a polymeric cationic substance.

**5.** The method according to Claim 4, wherein said polymeric cation is selected from the group consisting of diethylaminoethyldextran, diallyldimethylammonium chloride-hydroxyethyl cellulose polymer, and hexadimethrine bromide.

**6.** The method according to Claim 4, wherein said polymeric anionic molecule is selected from the group consisting of polyglutamic acid, anionic protein or derivatized proteins, anionic polysaccharides, polyaspartic acid, polyacrylic acid, and polyamino acids.

**7.** The method according to Claim 6, wherein said derivatized protein is derivatized albumin and wherein said anionic polysaccharide is heparin or alginic acid.

**Claims for the following Contracting State : AT**

1. A method for detecting an analyte in a test sample, comprising the steps of:
   a) contacting the sample with
   a soluble capture reagent, comprising a hapten conjugated to a first charged substance, and
   an indicator reagent, comprising a specific binding member conjugated to a label capable of producing a detectable signal, thereby forming a reaction mixture,
   wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,
   b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and
   c) detecting said label associated with said solid phase or said reaction mixture as an indication of the presence or amount of the analyte in the sample.

2. A method for detecting an analyte in a test sample, comprising the steps of:
   a) contacting the sample with
   a soluble capture reagent, comprising a specific binding member conjugated to a first charged substance, and
   an indicator reagent, comprising a hapten conjugated to a label capable of producing a detectable signal, thereby forming a reaction mixture,
   wherein said capture reagent is capable of directly binding a member selected from the group consisting of the analyte, said indicator reagent and a complex thereof or indirectly binding said member by binding to an ancillary specific binding member which is capable of binding to said member, and wherein said indicator reagent is capable of directly binding a member selected from the group consisting of the analyte, said capture reagent and a complex thereof or indirectly binding said member by binding to an ancillary specific binding member which is capable of binding to said member,
   b) contacting said reaction mixture with a solid phase having an opposite charge with respect to said first charged substance, whereby said solid phase attracts and attaches to said first charges substance, thereby enabling the separation of said capture reagent and complexes thereof from said reaction mixture; and
   c) detecting said label associated with said solid phase or said reaction mixture as an indication of the presence or amount of the analyte in the sample.

3. The method according to Claim 1 or 2, wherein a second charged substance having an opposite charge with respect to said first charged substance is retained on said solid phase material.

4. The method according to Claim 3, wherein said first charged substance is a polymeric anionic substance and said second charged substance is a polymeric cationic substance.

5. The method according to Claim 4, wherein said polymeric cation is selected from the group consisting of a diethylaminoethyldextran, diallyldimethylammonium chloride-hydroxyethyl cellulose polymer, and hexadimethrine bromide.

6. The method according to Claim 4, wherein said polymeric anionic molecule is selected from the group consisting of polyglutamic acid, anionic protein or derivatized proteins, anionic polysaccharides, polyaspartic acid, polyacrylic acid, and polyamino acids.

7. The method according to Claim 6, wherein said derivatized protein is derivatized albumin and wherein said anionic polysaccharide is heparin or alginic acid.

**Patentansprüche**
**Patentansprüche für folgende Vertragsstaaten : BE, CH, DE, ES, FR, GB, IT, LI, NL**

1. Verfahren zum Nachweis eines Analyten in einer Testprobe, das folgende Schritte umfaßt:
   a.) in-Kontakt-Bringen der Probe mit einem löslichen Fangreagens, das ein Hapten umfaßt, das an eine erste geladene Substanz konjugiert ist, und
   einem Indikatorreagens, das ein spezifisch bindendes Glied umfaßt, das an eine Markierung konjugiert ist, die zur Erzeugung eines nachweisbaren Signals befähigt ist, wodurch eine Reaktionsmischung gebildet wird,
   wobei das Fangreagens zur direkten Bindung eines Glieds befähigt ist, das aus der Gruppe gewählt ist, die aus dem Analyten, dem Indikatorreagens und einem Komplex daraus besteht, oder zur indirekten Bindung des Glieds, indem das Fangreagens an ein spezifisch bindendes Hilfsglied bindet, das zur Bindung des Glieds befähigt ist, und wobei das Indikatorreagens zur direkten Bindung eines Glieds befähigt ist, das aus der Gruppe gewählt ist, die aus dem Analyten, dem Fangreagens und einem Komplex daraus besteht, oder zur indirekten Bindung des Glieds, indem das Indikatorreagens an ein spezifisch bindendes Hilfsglied bindet, das zur Bindung des Glieds befähigt ist,
   b.) in-Kontakt-Bringen der Reaktionsmischung mit einer festen Phase, die in bezug auf die erste geladene Substanz entgegengesetzt geladen ist, wobei die feste Phase die erste geladene Substanz anzieht und festhält, wodurch die Abtrennung des Fangreagenzes und des Komplexes daraus von der Reaktionsmischung ermöglicht wird; und
   c.) Nachweis der Markierung, die mit der festen Phase oder der Reaktionsmischung assoziiert ist, als Anzeige für die Anwesenheit oder Menge des Analyten in der Probe.

2. Verfahren zum Nachweis eines Analyten in einer Testprobe, das folgende Schritte umfaßt:
   a.) in-Kontakt-Bringen der Probe mit einem löslichen Fangreagens, das ein spezifisches Bindungsglied umfaßt, das an eine erste geladene Substanz konjugiert ist, und
   einem Indikatorreagenz, das ein Hapten umfaßt, das an eine Markierung konjugiert ist, die zur Erzeugung eines nachweisbaren Signals befähigt ist, wodurch eine Reaktionsmischung gebildet wird,
   wobei das Fangreagens zur direkten Bindung eines Glieds befähigt ist, das aus der Gruppe gewählt ist, die aus dem Analyten, dem Indikatorreagens und einem Komplex daraus besteht, oder zur indirekten Bindung des Glieds, indem das Fangreagens an ein spezifisch bindendes Hilfsglied bindet, das zur Bindung des Glieds befähigt ist, und wobei das Indikatorreagens zur direkten Bindung eines Glieds befähigt ist, das aus der Gruppe gewählt ist, die aus dem Analyten, dem Fangreagens und einem Komplex daraus besteht, oder zur indirekten Bindung des Glieds, indem das Indikatorreagens an ein spezifisch bindendes Hilfsglied bindet, das zur Bindung des Glieds befähigt ist,
   b.) in-Kontakt-Bringen der Reaktionsmischung mit einer festen Phase, die in bezug auf die erste geladene Substanz entgegengesetzt geladen ist, wobei die feste Phase die erste geladene Substanz anzieht und festhält, wobei die Abtrennung des Fangreagenzes und des Komplexes daraus von der Reaktionsmischung ermöglicht wird; und
   c.) Nachweis der Markierung, die mit der festen Phase oder der Reaktionsmischung assoziiert ist, als Anzeige für die Anwesenheit oder Menge des Analyten in der Probe,
   vorausgesetzt, daß Konkurrenzassays, bei denen das Indikatorreagens und der Analyt bei der Bindung um das Fangreagens konkurrieren, ausgeschlossen sind.

3. Verfahren nach Anspruch 1 oder 2, wobei eine zweite geladene Substanz, die in bezug auf die erste geladene Substanz entgegengesetzt geladen ist, auf dem Festphasenmaterial zurückgehalten wird.

4. Verfahren nach Anspruch 3, wobei die erste geladene Substanz eine polymere anionische Substanz und die zweite geladene Substanz eine polymere kationische Substanz ist.

5. Verfahren nach Anspruch 4, wobei das polymere Kation aus der Gruppe gewählt ist, die aus Diethylaminoethyldextran, Diallyldimethylammoniumchlorid-Hydroxyethylcellulosepolymer und Hexadimethrinbromid besteht.

6. Verfahren nach Anspruch 4, wobei das polymere anionische Molekül aus der Gruppe gewählt ist, die aus Polyglutaminsäure, anionischem Protein oder derivatisierten Proteinen, anionischen Polysacchariden, Polyaspartamsäure, Polyacrylsäure und Polyaminosäuren besteht.

7. Verfahren nach Anspruch 6, wobei das derivatisierte Protein derivatisiertes Albumin ist, und wobei das anionische Polysaccharid Heparin oder Alginsäure ist.

**Patentansprüche für folgenden Vertragsstaat : AT**

1. Verfahren zum Nachweis eines Analyten in einer Testprobe, das folgende Schritte umfaßt:
   a.) in-Kontakt-Bringen der Probe mit einem löslichen Fangreagens, das ein Hapten umfaßt, das an eine erste geladene Substanz konjugiert ist, und

   einem Indikatorreagens, das ein spezifisch bindendes Glied umfaßt, das an eine Markierung konjugiert ist, die zur Erzeugung eines nachweisbaren Signals befähigt ist, wodurch eine Reaktionsmischung gebildet wird,

   wobei das Fangreagens zur direkten Bindung eines Glieds befähigt ist, das aus der Gruppe gewählt ist, die aus dem Analyten, dem Indikatorreagens und einem Komplex daraus besteht, oder zur indirekten Bindung des Glieds, indem das Fangreagens an ein spezifisch bindendes Hilfsglied bindet, das zur Bindung des Glieds befähigt ist, und wobei das Indikatorreagens zur direkten Bindunq eines Glieds befähigt ist, das aus der Gruppe gewählt ist, die aus dem Analyten, dem Fangreagens und einem Komplex daraus besteht, oder zur indirekten Bindung des Glieds, indem das Indikatorreagens an ein spezifisch bindendes Hilfsglied bindet, das zur Bindung des Glieds befähigt ist,

   b.) in-Kontakt-Bringen der Reaktionsmischung mit einer festen Phase, die in bezug auf die erste geladene Substanz entgegengesetzt geladen ist, wobei die feste Phase die erste geladene Substanz anzieht und festhält, wodurch die Abtrennung des Fangreagenzes und des Komplexes daraus von der Reaktionsmischung ermöglicht wird; und

   c.) Nachweis der Markierung, die mit der festen Phase oder der Reaktionsmischung assoziiert ist, als Anzeige für die Anwesenheit oder Menge des Analyten in der Probe.

2. Verfahren zum Nachweis eines Analyten in einer Testprobe, das folgende Schritte umfaßt:
   a.) in-Kontakt-Bringen der Probe mit einem löslichen Fangreagens, das ein spezifisches Bindungsglied umfaßt, das an eine erste geladene Substanz konjugiert ist, und

   einem Indikatorreagenz, das ein Hapten umfaßt, das an eine Markierung konjugiert ist, die zur Erzeugung eines nachweisbaren Signals befähigt ist, wodurch eine Reaktionsmischung gebildet wird,

   wobei das Fangreagens zur direkten Bindung eines Glieds befähigt ist, das aus der Gruppe gewählt ist, die aus dem Analyten, dem Indikatorreagens und einem Komplex daraus besteht, oder zur indirekten Bindung des Glieds, indem das Fangreagens an ein spezifisch bindendes Hilfsglied bindet, das zur Bindung des Glieds befähigt ist, und wobei das Indikatorreagens zur direkten Bindung eines Glieds befähigt ist, das aus der Gruppe gewählt ist, die aus dem Analyten, dem Fangreagens und einem Komplex daraus besteht, oder zur indirekten Bindung des Glieds, indem das Indikatorreagens an ein spezifisch bindendes Hilfsglied bindet, das zur Bindung des Glieds befähigt ist,

   b.) in-Kontakt-Bringen der Reaktionsmischung mit einer festen Phase, die in bezug auf die erste geladene Substanz entgegengesetzt geladen ist, wobei die feste Phase die erste geladene Substanz anzieht und festhält, wobei die Abtrennung des Fangreagenzes und des Komplexes daraus von der Reaktionsmischung ermöglicht wird; und

   c.) Nachweis der Markierung, die mit der festen Phase oder der Reaktionsmischung assoziiert ist, als Anzeige für die Anwesenheit oder Menge des Analyten in der Probe.

3. Verfahren nach Anspruch 1 oder 2, wobei eine zweite geladene Substanz, die in bezug auf die erste geladene Substanz entgegengesetzt geladen ist, auf dem Festphasenmaterial zurückgehalten wird.

4. Verfahren nach Anspruch 3, wobei die erste geladene Substanz eine polymere anionische Substanz und die zweite geladene Substanz eine polymere kationische Substanz ist.

5. Verfahren nach Anspruch 4, wobei das polymere Kation aus der Gruppe gewählt ist, die aus Diethylaminoethyldextran, Diallyldimethylammoniumchlorid-Hydroxyethylcellulosepolymer und Hexadimethrinbromid besteht.

6. Verfahren nach Anspruch 4, wobei das polymere anionische Molekül aus der Gruppe gewählt ist, die aus Polyglutaminsäure, anionischem Protein oder derivatisierten Proteinen, anionischen Polysacchariden, Polyaspartamsäure, Polyacrylsäure und Polyaminosäuren besteht.

7. Verfahren nach Anspruch 6, wobei das derivatisierte Protein derivatisiertes Albumin ist, und wobei das anionische Polysaccharid Heparin oder Alginsäure ist.

**Revendications**

**Revendications pour les Etats contractants suivants : BE, CH, DE, ES, FR, GB, IT, LI, NL**

1. Procédé de détection d'un analyte dis un échantillon à doser, comprenant les étapes consistant :

a) à mettre en contact l'échantillon avec

un réactif de capture soluble comprenant un haptène conjugué à une première substance chargée, et

un réactif d'indicateur comprenant un élément de liaison spécifique conjugué à un traceur capable de produire un signal détectable, formant par là un mélange de réaction,

dans lequel ledit réactif de capture est capable de se lier directement à un élément choisi parmi l'analyte, ledit réactif d'indicateur et un complexe de celui-ci ou de se lier indirectement audit élément en se liant à un élément de liaison spécifique auxiliaire qui est capable de se lier audit élément, et dans lequel ledit réactif d'indicateur est capable de se lier directement à un élément choisi parmi l'analyte, ledit réactif de capture et un complexe de celui-ci ou de se lier indirectement audit élément en se liant à un élément de liaison spécifique auxiliaire qui est capable de se lier audit élément,

b) à mettre en contact ledit mélange de réaction avec une phase solide présentant une charge opposée à celle de ladite première substance chargée, sur quoi ladite phase solide attire et se fixe à ladite première substance chargée, permettant par là la séparation dudit réactif de capture et des complexes de celui-ci dudit mélange de réaction ; et

c) à détecter ledit traceur associé à ladite phase solide ou audit mélange de réaction comme une indication de la présence ou de la quantité de l'analyte dans l'échantillon.

2. Procédé de détection d'un analyte dans un échantillon à doser, comprenant les étapes consistant :

a) à mettre en contact l'échantillon avec

un réactif de capture soluble comprenant un élément de liaison spécifique conjugué à une première substance chargée, et

un réactif d'indicateur comprenant un haptène conjugué à un traceur capable de produire un signal détectable, formant par là un mélange de réaction,

dans lequel ledit réactif de capture est capable de se lier directement à un élément choisi parmi l'analyte, ledit réactif d'indicateur et un complexe de celui-ci ou de se lier indirectement audit élément en se liant à un élément de liaison spécifique auxiliaire qui est capable de se lier audit élément, et dans lequel ledit réactif d'indicateur est capable de se lier directement à un élément choisi parmi l'analyte, ledit réactif de capture et un complexe de celui-ci ou de se lier indirectement audit élément en se liant à un élément de liaison spécifique auxiliaire qui est capable de se lier audit élément,

b) à mettre en contact ledit mélange de réaction avec une phase solide présentant une charge opposée à celle de ladite première substance chargée, sur quoi ladite phase solide attire et se fixe à ladite première substance chargée, permettant par là la séparation dudit réactif de capture et des complexes de celui-ci dudit mélange de réaction ; et

c) à détecter ledit traceur associé à ladite phase solide ou audit mélange de réaction comme une indication de la présence ou de la quantité de l'analyte dans l'échantillon,

à condition que soient exclus des dosages compétitifs dans lesquels le réactif d'indicateur et l'analyte sont en compétition pour se lier au réactif de capture.

3. Procédé selon la revendication 1 ou 2, dans lequel une seconde substance chargée présentant une charge opposée à celle de ladite première substance chargée est retenue sur ledit matériau de phase

solide.

4. Procédé selon la revendication 3, dans lequel ladite première substance chargée est une substance anionique polymère et ladite seconde substance chargée est une substance cationique polymère.

5. Procédé selon la revendication 4, dans lequel ledit cation polymère est choisi parmi le diéthylaminoéthyldextrane, le polymère de chlorure de diallyldiméthylammonium-hydroxyéthylcellulose et le bromure d'hexadiméthrine.

6. Procédé selon la revendication 4, dis lequel ladite molécule anionique polymère est choisie parmi l'acide polyglutamique, des protéines anioniques ou des protéines dérivées, des polysaccharides anioniques, l'acide polyaspartique, l'acide polyacrylique et des polyaminoacides.

7. Procédé selon la revendication 6, dans lequel ladite protéine dérivée est de l'albumine dérivée et dans lequel ledit polysaccharide anionique est l'héparine ou l'acide alginique.

**Revendications pour l'Etat contractant suivant : AT**

1. Procédé de détection d'un analyte dans un échantillon à doser, comprenant les étapes consistant :
   a) à mettre en contact l'échantillon avec
   un réactif de capture soluble comprenant un haptène conjugué à une première substance chargée, et
   un réactif d'indicateur comprenant un élément de liaison spécifique conjugué à un traceur capable de produire un signal détectable, formant par là un mélange de réaction,
   dans lequel ledit réactif de capture est capable de se lier directement à un élément choisi parmi l'analyte, ledit réactif d'indicateur et un complexe de celui-ci ou de se lier indirectement audit élément en se liant à un élément de liaison spécifique auxiliaire qui est capable de se lier audit élément, et dans lequel ledit réactif d'indicateur est capable de se lier directement à un élément choisi parmi l'analyte, ledit réactif de capture et un complexe de celui-ci ou de se lier indirectement audit élément en se liant à un élément de liaison spécifique auxiliaire qui est capable de se lier audit élément,
   b) à mettre en contact ledit mélange de réaction avec une phase solide présentant une charge opposée à celle de ladite première substance chargée, sur quoi ladite phase solide attire et se fixe à ladite première substance chargée, permettant par là la séparation dudit réactif de capture et des complexes de celui-ci dudit mélange de réaction ; et
   c) à détecter ledit traceur associé à ladite phase solide ou audit mélange de réaction comme une indication de la présence ou de la quantité de l'analyte dans l'échantillon.

2. Procédé de détection d'un analyte dans un échantillon à doser, comprenant les étapes consistant :
   a) à mettre en contact l'échantillon avec
   un réactif de capture soluble comprenant un élément de liaison spécifique conjugué à une première substance chargée, et
   un réactif d'indicateur comprenant un haptène conjugué à un marqueur capable de produire un signal détectable, formant par là un mélange de réaction,
   dans lequel ledit réactif de capture est capable de se lier directement à un élément choisi parmi l'analyte, ledit réactif d'indicateur et un complexe de celui-ci ou de se lier indirectement audit élément en se liant à un élément de liaison spécifique auxiliaire qui est capable de se lier audit élément, et dans lequel ledit réactif d'indicateur est capable de se lier directement à un élément choisi parmi l'analyte, ledit réactif de capture et un complexe de celui-ci ou de se lier indirectement audit élément en se liant à un élément de liaison spécifique auxiliaire qui est capable de se lier audit élément,
   b) à mettre en contact ledit mélange de réaction avec une phase solide présentant une charge opposée à celle de ladite première substance chargée, sur quoi ladite phase solide attire et se fixe à ladite première substance chargée, permettant par là la séparation dudit réactif de capture et des complexes de celui-ci dudit mélange de réaction ; et
   c) à détecter ledit traceur associé à ladite phase solide ou audit mélange de réaction comme une indication de la présence ou de la quantité de l'analyte dans l'échantillon.

**3.** Procédé selon la revendication 1 ou 2, dans lequel une seconde substance chargée présentant une charge opposée à celle de ladite première substance chargée est retenue sur ledit matériau de phase solide.

**4.** Procédé selon la revendication 3, dans lequel ladite première substance chargée est une substance anionique polymère et ladite seconde substance chargée est une substance cationique polymère.

**5.** Procédé selon la revendication 4, dans lequel ledit cation polymère est choisi parmi le diéthylaminoéthyldextrane, le polymère de chlorure de diallyldiméthylammonium-hydroxyéthylcellulose et le bromure d'hexadiméthrine.

**6.** Procédé selon la revendication 4, dans lequel ladite molécule anionique polymère est choisie parmi l'acide polyglutamique, des protéines anioniques ou des protéines dérivées, des polysaccharides anioniques, l'acide polyaspartique, l'acide polyacrylique et des polyaminoacides.

**7.** Procédé selon la revendication 6, dans lequel ladite protéine dérivée est de l'albumine dérivée et dans lequel ledit polysaccharide anionique est l'héparine ou l'acide alginique.